(19) Europäisches Patentamt
European Patent Office
Office européen des brevets

(11) **EP 4 346 620 B1**

(12) **EUROPEAN PATENT SPECIFICATION**

(45) Date of publication and mention
of the grant of the patent:
**07.05.2025 Bulletin 2025/19**

(21) Application number: **22730572.9**

(22) Date of filing: **01.06.2022**

(51) International Patent Classification (IPC):
*A61B 8/12* *(2006.01)*    *A61B 18/22* *(2006.01)*
*A61B 8/08* *(2006.01)*

(52) Cooperative Patent Classification (CPC):
**A61B 18/22; A61B 8/085; A61B 8/12;**
**A61B 8/4209; A61B 8/4218; A61B 8/4488;**
**A61B 8/5238; A61B 8/5261;** A61B 2017/3411;
A61B 2018/00547; A61B 2018/00577;
A61B 2018/2005; A61B 2034/104; A61B 2034/2059;
A61B 2090/3782

(86) International application number:
**PCT/EP2022/064965**

(87) International publication number:
**WO 2022/253920 (08.12.2022 Gazette 2022/49)**

(54) **APPARATUS FOR THE EMISSION OF TUMOR CELL DESTRUCTIVE RADIATION**

VORRICHTUNG ZUR EMISSION VON TUMORZELLZERSTÖRENDER STRAHLUNG

APPAREIL POUR L'ÉMISSION D'UN RAYONNEMENT DESTRUCTEUR DE CELLULES TUMORALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2021 IT 202100014654**

(43) Date of publication of application:
**10.04.2024 Bulletin 2024/15**

(73) Proprietor: **ELESTA S.P.A.**
**50041 Calenzano (FI) (IT)**

(72) Inventors:
• **BRESCHI, Luca**
**59021 Vaiano (PO) (IT)**

• **ANDREOLA, Fabio**
**50041 Calenzano (FI) (IT)**
• **MASOTTI, Giovanni**
**50041 Calenzano (FI) (IT)**

(74) Representative: **Mannucci, Michele et al**
**Ufficio Tecnico**
**Ing. A. Mannucci S.r.l.**
**Via della Scala, 4**
**50123 Firenze (IT)**

(56) References cited:
WO-A1-2020/212893    US-A1- 2005 124 884
US-A1- 2010 016 710    US-A1- 2011 071 380
US-A1- 2016 199 668    US-A1- 2017 020 623
US-A1- 2020 108 222

# EP 4 346 620 B1

## Description

### Technical field

The present invention concerns medical equipment and its methods of use. More specifically, it describes innovations in the sector of equipment in the field of surgical interventions, in particular, but not exclusively, on the prostate.

### State of the art

The aim of FLA (Focal Laser Ablation) of the prostate is to thermally destroy a unifocal malignant tumour carcinoma. In this context, reference is made to document US2017/0020623 A1.

The current interventional techniques entail monitoring of the intervention area by means of imaging techniques such as, for example, ultrasound imaging, displaying on a screen, during the intervention, the positioning of laser optical fibres which are introduced into the intervention area by guide needles passed through the perineum of the patient.

One objective of said surgical technique is to remove all the tumour, leaving the fundamental anatomical structures around it intact. There is therefore the need to destroy portions of anatomical structure around the tumour to avoid leaving the slightest trace of tumour cells in the structure in question, but it is also necessary not to eliminate too much of the anatomical structure, in order to allow functional conservation of the organ involved and the adjacent structures.

### Summary

The object of the present invention is therefore to improve the aspects connected with interventions for the removal of tumours, and in particular prostate tumours, developing an apparatus and a method for improving the precision of the intervention.

The invention is defined in the appended claims. Methods of therapeutic or surgical treatment of a living human tissue mentioned hereinafter do not form part of the present invention. An important object of the present invention is, for example, to provide an apparatus for the emission of tumour cell destructive radiation that allows only the pre-selected anatomical structures to be destroyed.

A further object of the present invention is, for example, to provide an apparatus for the emission of tumour cell destructive radiation that allows adequate planning of the areas to be destroyed.

Last but not least, a further object of the present invention is to develop an image processing method that allows the outcome of an intervention for removal of tumour cells, for example an intervention on the prostate, to be planned and predicted.

These and other objects, which will become clearer below, are achieved by an apparatus for the emission of tumour cell destructive radiation, comprising

- an ultrasonic probe configured to acquire at least one first image relating to a spatial volume where the tumour cells to be destroyed are present,
- a first support system, configured to support the ultrasonic probe in the area to be investigated, the position of said ultrasonic probe in relation to the first support system being known,
- at least one tumour cell destructive radiation emission head,
- a second support system, configured to support and guide, namely allow to travel a given trajectory, the at least one emission head in, or in the vicinity of, the area with the tumour cells to be destroyed, said second support system being in spatial relationship with the first support system whereby the position of the second support system is known, namely the position of the given trajectory of said at least one emission head, with respect to the ultrasonic probe, and with respect to the spatial volume investigated by the ultrasonic probe,
- an electronic device for managing the apparatus, comprising a screen and an electronic program adapted to combine at least one said first image deriving from the acquisition of the ultrasonic probe with at least one second diagnostic image different from images acquired by the ultrasonic endocavitary probe and relating to a volume of the same area where the tumour cells to be destroyed are present, in order to obtain at least a third combined image, and wherein the at least one third combined image is adapted to be shown on said screen;

the electronic program comprises a module for simulating the destruction of the tumour cells which entails the operations of

spatial definition, on at least said third combined image shown on the screen, of the intervention area where the tumour cell destructive radiation is to be emitted,

display, on said at least one third combined image shown on the screen, of a simulation of the position of at least part of said second support system, namely of at least part of said given trajectory, whereby it is possible to virtually position at least one said emission head in said intervention area,

virtual positioning of said at least one said emission head in said intervention area shown on the screen in said at least one third combined image,

display, on said at least one third combined image shown on the screen, of a simulation of the volume that can be irradiated by said at least one emission head virtually positioned in said intervention area.

As said, the apparatus according to the invention lends itself well to the focal treatment of unifocal tumours of the prostate by laser ablation. Therefore, the ultrasonic probe is preferably an endocavitary probe, for example of the transrectal or endorectal type, e.g. a transrectal or endorectal ultrasound probe. Furthermore, preferably, the at least one tumour cell destructive radiation emission head is a member adapted to emit a laser beam, preferably the end of an optical fibre. The apparatus therefore also comprises a laser light source with which the optical fibre is associated.

Preferably, the at least one tumour cell destructive radiation emission head is arranged inside a guide needle movable by means of the second guide system.

The apparatus also lends itself well to the treatment of tumours in other areas of the body such as, for example, the abdomen.

Preferably, the electronic program of the electronic device for managing the apparatus comprises different operating modules, including an image combination module, adapted to combine first images deriving from the acquisition of the ultrasound probe with second diagnostic images different from images acquired by the ultrasonic probe and relating to the same area where the tumour cells to be destroyed are present, in order to obtain third combined images that can be displayed on the screen, a module for planning/simulating the destruction of the tumour cells and a module relating to the actual intervention for destruction of the tumour cells based on the simulations of the preceding simulation/planning module.

As regards the combination of images, preferably the at least one second diagnostic image to be combined with the at least one first image acquired by the ultrasonic probe is different from images deriving from ultrasonic probes and more preferably is an image deriving from a magnetic resonance, preferably in high definition.

Said need to combine different image types derives from the fact that often the images deriving from ultrasound inspection are not able to clearly highlight the tumour area and it is therefore necessary to compensate for the lack of information by means of images from other diagnostic examinations such as, for example, magnetic resonance imaging.

In practice, at the moment of combination of the images (a technique generally known as fusion), a set of images of the tumour area to be treated must be available in order to combine them with the images from the ultrasonic probe that monitors the treatment area during the intervention.

The ultrasonic probe shows images of the ultrasonic volume investigated in real time; these images can be three-dimensional or flat images, for example according to the sagittal, coronal and transverse planes (which, when combined, allow a three-dimensional image to be reconstructed).

Analogously, magnetic resonance shows two-dimensional images with respect, for example, to the same sagittal, coronal and transverse planes and/or a three-dimensional combination of two or three of them.

Therefore, preferably, the electronic program of the apparatus is able to manage both several first images of different planes and one or more first three-dimensional images. Analogously the electronic program of the apparatus is able to manage both several second images of different planes, and one or more second three-dimensional images. For example, the first two-dimensional images of respective planes can be combined with the second two-dimensional images of corresponding sagittal, coronal and transverse planes.

Preferably, the at least one second image is stored in the electronic management device before acquisition of the at least one first image by the ultrasonic probe.

For example, the patient is scanned in the area in question by magnetic resonance and therefore magnetic resonance volumes are acquired in which the tumour area is present. The files with the image or images of the magnetic resonance (the co-called "second image") are acquired in the apparatus according to the invention.

For example, the ultrasonic probe is inserted in the rectum of the patient. This probe is associated with a said first support system which is preferably also configured to guide the ultrasonic probe into the rectum. Expediently, the first support system allows the position of the ultrasonic probe in the first support system, namely in space, to be always known. For example, the probe is associated with a reference system which is fixed with respect to the patient, for example fixed with respect to the patient examination table, namely with respect to a probe support which is fixed with respect to the examination table on which the patient is lying still.

Once the probe has allowed the acquisition of an ultrasonic volume of the area to be treated, which translates into the acquisition of at least one first image (for example at least three two-dimensional images relating for example to the sagittal, coronal or transverse plane, or a combination of the three planes to create at least a first three-dimensional image, or directly a three-dimensional image), in this example of the prostate, the electronic management device, by means of the above-mentioned electronic program, produces the combination (or fusion) of said at least one first and at least one second image, so that the at least one third image resulting from the combination shows both the volume of the area to be treated acquired in real time from the patient by means of the ultrasonic probe, and the area to be treated showing the tumour cells to be destroyed.

Preferably, the combination of the at least one first and the at least one second image is obtained by identifying reference points or areas relative to the same physical points of the patient both on the at least one first and on the at least one second image and trying to position the images so that these points or areas are as near as possible. They can be points (for example at least three points) or non-puntiform portions or anatomical structures such as, for example, the non-rectilinear course of the urethra or the edge of the prostate gland in various planes (in the case of the treatment of a prostate tumour).

Said combination can be obtained for example by means of static combination of the images, for example by simply fusing the first and second image, making the reference points or areas correspond as described above.

Or, said combination can be obtained for example by means of an elastic combination of the images, for example by superimposing the first and the second image, making the reference points or areas correspond as described above and then stretching, deforming, roto-translating, the second image (or the first image) to make the reference points or areas used for the combination correspond as closely as possible.

In general, fusion techniques of magnetic resonance and ultrasound images are known.

Preferably, the processing program provides that, before the combination of said at least one first and at least one second image, the second image shows a circumscription of the area occupied by the tumour cells, namely the definition, in the image, of a closed figure that follows the outline of the tumour visible in the image so that said circumscription of the area with the tumour cells is also found in said at least one third combined image.

For example, the processing program provides for an operation of circumscription of the area with the tumour cells before combination of the at least one first and at least one second image. For example, the second images, for example magnetic resonance images, can have been previously evaluated by a doctor who has identified the tumour area and has carried out on the images (namely on the files showing the images) a graphic circumscription (by means of an electronic program associated with the magnetic resonance machine or examination of the magnetic resonance results). For example, the area with the tumour can be circumscribed by the doctor with closed figures in the various planes, and therefore it assumes the connotation of a volume defined and positioned spatially in known coordinates in the set of volumetric images provided by the magnetic resonance.

In other examples, the circumscription of the tumour area can be carried out also after combination of the images to create the at least one third combined image.

Said at least one third combined image can be seen by the surgeon on a screen.

As said, the apparatus provides for a second support system for the at least one tumour cell destructive radiation emission head, for example an optical fibre adapted to emit laser light. Said second support system is configured to guide the at least one emission head along a given trajectory defined for example by the support itself in, or in the vicinity of, the area with the tumour cells to be destroyed. Expediently, said second support system, namely said given trajectory defined by the guide defined by the support system, is in spatial relationship with the first support system whereby the position of the second support system, namely of the given trajectory with respect to the ultrasonic probe, and with respect to the spatial volume investigated by the ultrasonic probe, is known.

For example, the second support system defines guide trajectories, the spatial development of which is known in the spatial reference system of the first support system in which the position of the ultrasonic probe is known. Therefore, in the at least one first image the position of the second support system, namely the position of the trajectory, is virtually defined, in other words the positions that said at least one emission head can assume in said first image are virtually known. This information is shown in the at least one third combined image, and therefore the support system, or more precisely the virtual extension of the trajectory defined by the support system in the treatment area can be seen on the surgeon's screen, therefore allowing simulation of the treatment for destruction of the tumour circumscribed in the at least one third combined image.

Preferably, the definition of the area of intervention also provides for the definition of a safety area not to be invaded by the destructive treatment.

Preferably, the dimension of the volume that can be irradiated by the at least one emission head virtually positioned in the intervention area is a function of one or more of the treatment parameters, including the power of the radiation emitted, the quantity of radiation emitted, any particular movement of the head during the emission phase etc.

For example, in the case of laser light, the dimension of the volume that can be irradiated by the at least one emission head virtually positioned in the intervention area is a function of one or more of the treatment parameters: power of the radiation emitted by the radiation source, quantity or dose of energy emitted, number of pullbacks of the emission head from the treatment area outwards, length of the pullbacks.

A pullback is a movement of the emission head or, in the case of a needle with optical fibre, a backward movement of the needle from the treatment area (extracting the needle from the patient) with subsequent emission of the laser light as soon as the pullback of the needle with the fibre has been effected. This action creates a particular form of the ablation volume in the treatment area which grows in an inverse direction to the direction of insertion of the needles. By length of the pullbacks we mean the distance, for example in mm, of movement from the treatment area backwards during the pullback.

Obviously, the parameters to be set also include the number of emission heads (with relative parameters) inserted simultaneously in the area to be treated.

The heads can be arranged and act all together in the area to be treated or be inserted according to a given sequence (and act according to a given sequence). Obviously also the positions of said heads contribute to forming the process parameters.

The surgeon for example chooses the work parameters and the position of the emission head and verifies that the intervention area is completely covered by the ablation volume emitted by the head. If the area is completely covered, without the ablation volume overstepping any safety area or volume, namely without affecting anatomical structures not to be damaged, then the surgeon decides that the simulation has been successful and can proceed with the treatment. If not, the surgeon will change the above-mentioned work parameters, including the position and/or number of emission heads used (one or more).

In preferred embodiments, the simulation module provides for real time calculation of the simulation based on the above-mentioned parameters, namely: number of ablation heads, mutual positioning of the ablation heads, energy dose applied by each ablation head, power of the ablation radiation source, any presence of pullback actions, with possible definition of the pullback length/distance.

In preferred embodiments, the simulation model comprises a database of previous simulations carried out based on the above-mentioned parameters, namely: number of ablation heads, mutual positioning of the ablation heads, energy dose applied by each ablation head, power of the ablation radiation source, any presence of pullback actions, with possible definition of the pullback length/distance; therefore the surgeon, instead of performing a direct simulation, with the calculation times determined by the simulation, can recover the results of a simulation already carried out previously with the same boundary conditions, given the same work parameters as those indicated above, thus saving on calculation time.

Expediently, the electronic program can comprise a module for beginning of the destructive treatment of the tumour cell area, which entails the setting of one or more treatment parameters, corresponding to the virtual treatment parameters set in the apparatus during operation of the simulation module; said parameters are for example the power of the radiation emitted, the quantity of radiation emitted, any particular movement of the head during the emission phase etc.. In the case of laser light, said treatment parameters are: power of the laser light emitted by the laser light source, quantity or dose of laser light energy emitted, number of pullbacks of the emission head from the treatment area outwards, length of the pullbacks.

According to preferred embodiments, the first support system (and guide) for the ultrasonic probe comprises a support device for example positioned on a carriage, which can comprise a slide or a complex of slides, on which the ultrasonic probe can be applied. Therefore, it is possible to know, for example by means of encoder systems or similar, the position of the carriage or of the ultrasonic probe in the slide complex. Preferably the support device can be fixed to an examination table where the patient is positioned, or in any case can be in a fixed position with respect to the examination table with the patient. Preferably, the slide complex can provide one or more of the following degrees of freedom (with knowledge of the position with respect to one or more of said degrees of freedom, in a reference system for example integral with the carriage/patient examination table): adjustment of height from ground; translation in a horizontal direction transversely to the edge of the examination table with which the carriage/device is associated; adjustment of the inclination of the ultrasonic probe around a vertical axis and around a horizontal axis, to align the longitudinal axis of the ultrasonic probe for example with the axis of the rectum of the patient; adjustment of the distance of the end of the ultrasonic probe for example from the anus of the patient, rotation of the probe around its longitudinal axis.

The support device for the ultrasonic probe, preferably the carriage, can comprise a constraint system, for example a cradle, which allows the probe to rotate around its longitudinal axis of development.

According to preferred embodiments, the ultrasonic probe comprises an oblong body, with a convex curved outer surface, extending along a longitudinal development of said body, in which on the body a plurality of ultrasonic sensors are provided facing the curved surface to emit and receive ultrasonic waves.

According to an embodiment, the ultrasonic sensors of the probe are arranged according to at least one rectilinear array, parallel to the probe axis and at least one curvilinear array, lying on a circumference on a plane orthogonal to the probe axis.

According to an embodiment, the ultrasonic sensors of the probe are arranged according to a two-dimensional matrix; preferably said two-dimensional matrix has a first dimension parallel to the longitudinal development of the ultrasonic probe body and a second dimension substantially orthogonal to the first dimension; preferably the curved surface of said ultrasonic probe is substantially cylindrical and has a longitudinal axis parallel to the longitudinal development of the oblong body, and in which the ultrasonic sensors are aligned according to a plurality of lines parallel to one another and parallel to the longitudinal axis of the cylindrical surface.

According to an embodiment, the ultrasonic sensors of the probe are arranged according to a single rectilinear array parallel to the probe axis, and a single curvilinear array lying on a circumference on a plane orthogonal to the probe axis.

Preferably, the apparatus provided with one of the ultrasonic probe configurations just defined comprises a device for controlling switching of the ultrasonic sensors of said ultrasonic probe; preferably said device for controlling switching of the ultrasonic sensors being configured to sequentially activate ultrasonic sensors belonging to lines parallel to the longitudinal axis of the consecutive cylindrical surface, to acquire a sequence of ultrasound images according to a plurality of angularly offset scanning planes passing through the longitudinal axis of the cylindrical surface and angularly offset from one another; preferably said ultrasonic sensor switching control device being configured to sequentially activate ultrasonic sensors belonging to consecutive circumferential lines, to acquire ultrasound images according to a plurality of scanning planes orthogonal to the longitudinal axis of the cylindrical surface and offset along said longitudinal axis; preferably said ultrasonic sensor switching control device being configured to simultaneously activate at least one linear array of ultrasonic sensors angularly offset from one another around the longitudinal axis and linearly along the longitudinal axis of the cylindrical surface to acquire an image according to an oblique plane with respect to the longitudinal axis of the cylindrical surface.

In preferred embodiments, the second support system that allows said at least one emission head to be guided along a given trajectory in, or in the vicinity of, the area with the tumour cells to be destroyed is for example fixed to the support device that comprises the first support system for the ultrasonic probe. In this way the position of the second support system, and the development, namely the trajectory, of the guide or guides for said head is known in the reference system associated with said support device, namely a reference system in which the position of the ultrasonic probe is known.

Preferably, the second support system comprises a guide member that defines a matrix of guides, for example parallel to one another, such as for example through channels, in which the emission heads can run in parallel directions, distributed in a matrix, for example needles transporting optical fibres for the emission of laser light. Said through channels define the guide trajectories of the emission heads, and said trajectories are thus known with respect to a reference system that contains the guide member.

For example, this guide member with guide matrix can have fixed orientation with respect to the first ultrasonic probe support system, whereby the spatial relationship between the guides of the guide member and the position of the ultrasonic probe can be known. In other embodiments, said guide member can be associated with adjustment means for adjusting its spatial orientation, with associated means designed to identify the variation in spatial orientation with respect to the first support system, and in which said orientation variations are transmitted to the apparatus, in order that the position and relative orientation of the guide member (and therefore of the guides/trajectories defined by the latter), with respect to the first support system, or with respect to the known position of the ultrasonic probe, are always known. The variation in orientation of the guide member of the emission heads allows for example the operator to vary the possible position of the heads in the treatment area if a given orientation of said guides does not allow optimal treatment.

According to another aspect, the invention concerns a method of processing diagnostic images, including at least one first image acquired by means of an ultrasonic probe and at least a second image acquired by means of an imaging technique different from the one for acquisition by means of said ultrasonic probe, to simulate the emission of tumour cell destructive radiation, comprising the following steps:

- acquiring at least one said second image showing the area with the tumour cells to be treated,
- acquiring at least one said first image,
- associating with said at least one first image a virtual positioning structure for at least one tumour cell destructive radiation virtual emission head,
- combining said at least one said first image with said at least one second image to produce at least a third combined image, showing said area with the tumour cells and said virtual positioning structure of the virtual emission head,
- in said third combined image, a form of circumscription of the intervention area where the tumour cell destructive radiation is to be emitted can be seen,
- on said third combined image, virtually positioning said virtual emission head on said virtual positioning structure so as to virtually emit the destructive radiation in said intervention area,
- verifying that the volume that can be irradiated by said at least one virtual emission head covers the entire intervention area.

Preferably, in the third combined image, the method entails displaying said volume that can be irradiated by said at least one virtual emission head positioned in said intervention area.

Preferably, in the third combined image, a form of circumscription of a safety area surrounding the intervention area can be seen.

Preferably, the virtual positioning structure is positioned in said at least one image by means of a unique positioning relationship, so that given said image, said positioning structure can assume only the position defined by said unique positioning relationship.

Preferably, the display of said volume that can be irradiated by said at least one virtual emission head virtually positioned in said intervention area provides for

- real time calculation of the simulation based on one or more of the following parameters: number of ablation heads, mutual positioning of the ablation heads, energy dose applied by each ablation head, power of the ablation radiation source, presence of any pullback actions, with possible definition of the pullback length/distance, or
- a database of previous simulations carried out on the basis of one or more of the following parameters: number of ablation heads, mutual positioning of the ablation heads, dose of energy applied by each ablation head, power of the ablation radiation source, presence of any pullback actions, with possible definition of the pullback length/distance, whereby the surgeon can recover the results of a simulation already carried out previously with the same work parameters as those indicated above, saving on calculation time.

## Brief description of the drawings

The invention will be better understood by following the description and the attached drawings, which illustrate an embodiment of a non-limiting example of the invention. More in particular, the drawings show:

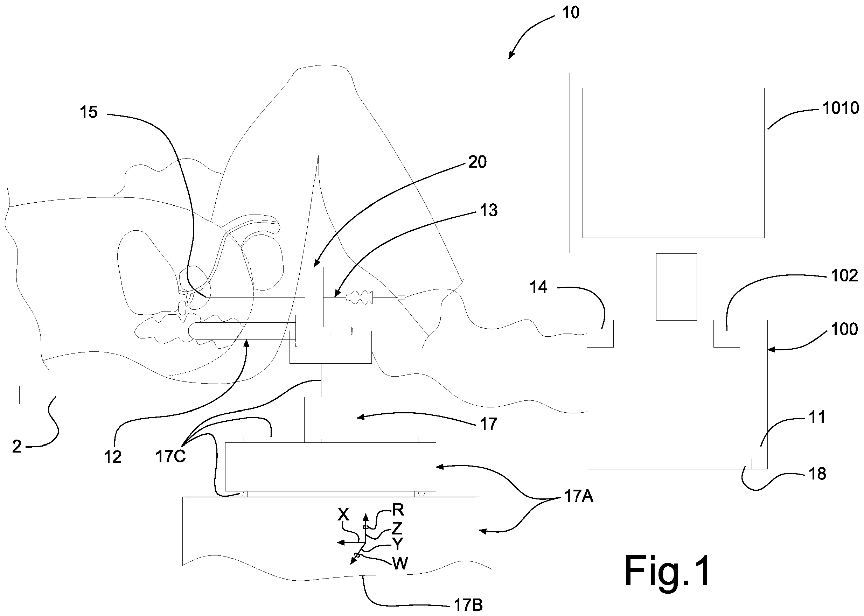

Fig. 1 a schematic view of the apparatus according to the invention, during treatment on a patient shown according to a median or sagittal plane;

Fig. 2 shows an example of a first ultrasonic probe that can be used in the apparatus according to the invention;

Fig. 3 shows an example of a second ultrasonic probe that can be used in the apparatus according to the invention;

Fig. 4 shows a guide member for a guide needle of an optical fibre which acts as a laser light emission head, for use in the apparatus according to the invention;

Fig 4A shows a schematic view of the enlargement of the end of the needle of figure 4;

Fig 4B shows a schematic view of a guide member, a variation with respect to that of figure 4;

Fig. 5 shows a diagram of an ultrasound image according to a transverse plane of an area of a patient with the prostate highlighted, carried out with a probe of figure 2 or 3 and with the apparatus according to the invention;

Fig. 6 shows a diagram of magnetic resonance according to a transverse plane of an area of a patient highlighting the prostate and a tumour area of the prostate;

Fig. 7 shows a portion of the magnetic resonance image of figure 6 referring only to the prostate;

Fig. 8 shows a portion of the ultrasound image of figure 5, with a portion of ultrasonic probe highlighted;

Fig. 9 shows a superimposition of the magnetic resonance and ultrasound images of figures 7 and 8, to create a combination of the two images in order to produce a third combined image, according to a fusion technique;

Fig. 10 shows a stretching of the magnetic resonance image of figure 9, for combination with the ultrasound image, in order to produce a combination of the two images according to an elastic fusion technique;

Fig. 11 shows a third image (again according to the transverse plane) produced by the combination highlighted in figures 7 to 10, in which the prostate and the tumour area are highlighted, and a guide matrix for optical fibre needles and areas of intervention and safety of the treatment around the tumour area;

Fig. 12 shows the same third image as figure 11 seen in relation to a median or sagittal plane;

Fig. 13 shows the same third image as figure 11 seen in relation to a frontal or coronal plane;

Fig. 14 to 16 show the respective images of figures 11 to 13, highlighting a needle with optical fibre designed to emit laser light and the ablation volume emitted;

Fig. 17 shows the same third image as figure 11 highlighting a plurality of ablation volumes that can be emitted by the laser emission head;

Fig. 18 shows a diagram of the phases of a method according to the invention, applied for example to the apparatus of the preceding figures;

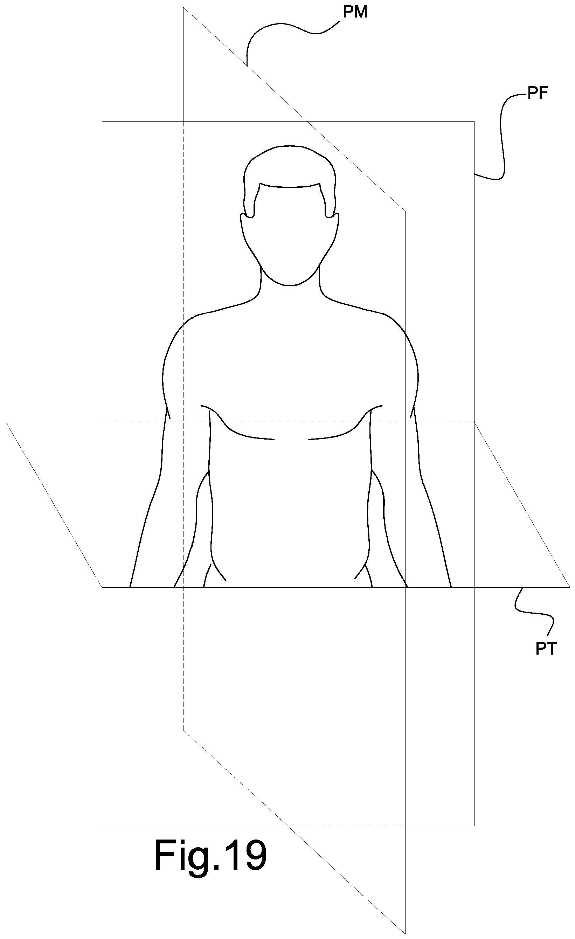

Fig. 19 shows the definition of the transverse plane PT, median/sagittal plane PM, frontal/coronal plane PF, relative to possible planes of two-dimensional images of diagnostic imaging.

## Detailed disclosure of embodiments

With reference to the figures previously cited, a tumour cell destructive radiation emission apparatus according to the invention is indicated overall by the number 10. In this example said apparatus is relative to the ablation treatment of a prostate tumour.

Said apparatus 10 comprises an ultrasound scanner 11 equipped with an ultrasonic probe 12, such as an endocavitary probe, preferably transrectal or endorectal, able to acquire images relative to a spatial volume around the prostate.

The apparatus further comprises a laser emission device 13 which provides a laser light source 14 operatively connected to one or more laser light emission heads 15, said light acting as a tumour cell destructive radiation, namely able to perform ablation of the tumour area.

For example, an emission head 15 is the end of an optical fibre 15A operatively connected to the laser light source 14, and arranged inside a guide needle 16, in turn positionable in the treatment area, as subsequently explained in further detail.

The apparatus 10 comprises an electronic management device, indicated overall by 100.

Returning to the ultrasonic endocavitary probe 12, it is associated with a first support system, configured to support said probe in the area to be investigated. Said first support system 17 is such that the position of the probe in the support system is known and, more in general, the position of the ultrasonic volume generated (namely the spatial volume investigated in the patient) is known in a reference system associated with the first support system, so that by moving the probe with respect to the support, the new position of the probe and the ultrasonic volume generated is known. In this example, said first support system 17 is also a guide system for the probe 12.

For example, the first support (and guide) system 17 for the endocavitary probe 12 comprises a support device 17A which comprises for example a carriage 17B which can be equipped with a slide or a complex of slides 17C, on which the ultrasonic probe 17 can be applied, and which allow the guided movement thereof into the rectum of the patient. Therefore, it is possible to know, for example by means of encoder systems or similar, the position of the carriage, namely of the endocavitary probe in the slide complex.

The support device 17A can be fixed to the examination table L where the patient is positioned, or in any case can be in a fixed position with respect to the examination table with the patient.

The slide complex 17C can provide the following degrees of freedom: adjustment Z of height from ground; translation Y in horizontal direction transversely to the edge of the examination table with which the carriage is associated, adjustment of the ultrasonic probe inclination around a vertical axis R and around a horizontal axis W, to align the longitudinal axis of the ultrasonic probe for example with the axis of the rectum of the patient; adjustment X of the distance of the end of the ultrasonic probe for example from the anus of the patient.

The ultrasonic endocavitary probe 12 is a volumetric probe, namely able to acquire images of an ultrasonic volume generated by the probe. The volume can be generated by the probe at a standstill in the rectum, or by a movement of the probe in the rectum, according to the type of probe used.

For example, in this embodiment, the ultrasonic probe used is one of those described in the international patent application WO2020/212893.

Therefore, in this example, the ultrasonic probe 12 comprises an oblong body 12A, with a convex curved outer surface, extending along a longitudinal development according to the axis Q of said body, in which a plurality of ultrasonic sensors 12B facing the curved surface is provided on the body, to emit and receive ultrasonic waves, arranged according to a combination of at least one rectilinear array parallel to the axis of the probe, and at least one curvilinear array lying on a circumference on a plane orthogonal to the axis of the probe.

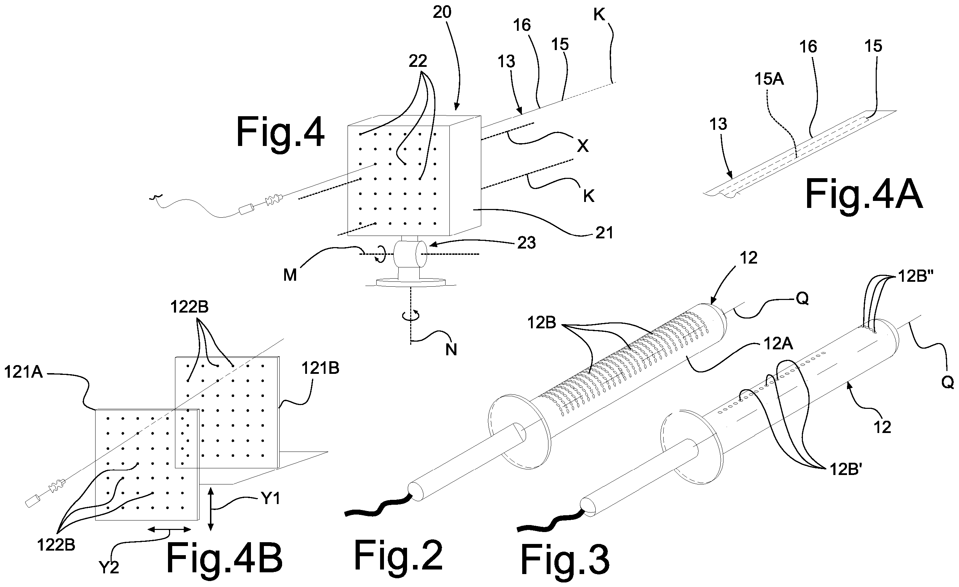

For example, as in figure 2, said ultrasonic sensors 12B are arranged according to a two-dimensional matrix that has a first dimension parallel to the longitudinal development of the body of the ultrasonic probe and a second dimension substantially orthogonal to the first dimension.

The curved surface of the ultrasonic probe 12 is substantially cylindrical and has a longitudinal axis parallel to the longitudinal development of the oblong body, and in which the ultrasonic sensors 12B are aligned according to a plurality of lines parallel to one another and parallel to the longitudinal axis of the cylindrical surface.

Again, as described in WO/2020/212893, the apparatus provided with the ultrasonic probe configuration just defined comprises, for example inside the ultrasound scanner 11, a control device 18 for controlling switching of the ultrasonic sensors 12B of the ultrasonic probe. For example, said device 18 for controlling switching of the ultrasonic sensors is configured to sequentially activate ultrasonic sensors belonging to lines parallel to the longitudinal axis of the consecutive cylindrical surface, to acquire a sequence of ultrasound images according to a plurality of angularly offset scanning planes passing through the longitudinal axis of the cylindrical surface and angularly offset from one another. For example, the device 18 is configured to sequentially activate ultrasonic sensors 12B belonging to consecutive circumferential lines, to acquire ultrasound images according to a plurality of scanning planes orthogonal to the longitudinal axis of the cylindrical surface and offset along said longitudinal axis.

Again, the control device 18 for controlling switching of the ultrasonic sensors 12B can be configured to simultaneously activate at least one linear array of ultrasonic sensors angularly offset from one another around the longitudinal axis and linearly along the longitudinal axis of the cylindrical surface to acquire an image according to an oblique plane with respect to the longitudinal axis of the cylindrical surface.

Figure 3 shows the case of an ultrasonic probe with one single rectilinear array 12B' parallel to the axis of the probe and one single curvilinear array 12B'' lying on a circumference on a plane orthogonal to the axis of the probe.

Obviously other types of ultrasonic transrectal/endorectal probes can be used able to scan ultrasonic volumes in the patient's body, by insertion into the rectum of the patient. For example, other probes in the form of oblong bodies can have ultrasonic sensors at the top of the end of the body, roughly in line with the body.

The probes indicated require a relative movement with respect to the area to be investigated, since the ultrasonic volume is generated by combination of the sensor front with the movement of the probe. In other examples, ultrasonic endocavitary probes can be used in which the body containing the ultrasonic sensors stays still with respect to the area to be investigated, while the sensors move over the body, in order to generate the ultrasonic volume.

The apparatus 10 further comprises a second support system 20, configured to support and guide at least one laser light emission head 15 (or several heads 15), along a given trajectory K (or several trajectories K) in, or in the vicinity of, the area with the tumour cells to be destroyed.

For example, the second support system 20 comprises a guide member 21 that defines a matrix of guides, for example parallel to one another such as, for example, through channels 22, in which the emission heads, and more in particular the needles 16 transporting the optical fibres, the ends of which form the emission heads 15, can run in parallel directions, distributed in a matrix. Said through channels 22 define the guide trajectories K of the needles 16 and therefore of the emission heads 15 contained in them.

Said second support system 20 is in spatial relationship with the first support system of the ultrasonic endocavitary probe, so that the position of the trajectories K defined by the guide member 21 is known in a reference system in which also the position of the endocavitary probe 12 is known (namely the spatial volume investigated by said endocavitary probe is known).

For example, the guide member 21 is constrained in a known manner to the support device 17A and oriented so that the through channels 22 guiding the needles 16 are oriented towards the perineum of the patient, so that the projection of the guide matrix covers the area of the prostate, hence a movement of the needles within the perineum brings them, and therefore the laser light emission heads, into the treatment area.

The guide member 21 is associated with an adjustment device 23 for adjusting the spatial orientation of the guides 22, associated with one or more systems 23A adapted to identify the variation in spatial orientation with respect to the first support system to which it is fixed, and in which said orientation variations are transmitted to the apparatus 100, so that the position and relative orientation of the guide member 21 (and therefore of the guides/trajectories K defined by the latter) with respect to the support device 17A are always known.

For example, said adjustment device 23 for adjusting the spatial orientation of the guides 22 provides for a hinge connecting to the support device 17A with a locking device for locking the member 21 in the desired position with respect to the support device. Expediently, an encoder system 23 associated with the rotation of the guide member around the hinge axis (or axes) allows identification of the variation in spatial orientation of the guide member, namely of the trajectories K associated with it. The constraint hinge can regulate the orientation of the guide member also around several axes, for example two axes N and M (or three axes) orthogonal to each other.

Another example of adjustment device for adjusting the spatial orientation of the guides 22 is provided in the example of figure 4B; in this case, the guide member comprises at least two matrixes 121A, 121B with through guide channels 122A-122B arranged on two facing surfaces, with the matrixes of the channels not rigidly aligned but having at least two degrees of freedom Y1, Y2; in particular the proximal one 121A can be moved up/down and left/right while the distal one 121B stays still on the perineum, whereby a needle changes inclination maintaining its height in the matrix 121B and varying it in the matrix 121A. For example, the through channels 122A and 122B have a clearance larger than the section of the needles that are guided inside them. The relative movements can be obtained with verniers and encoders that transfer to the system the amount of the movement. In this way they can generate known oblique trajectories.

Again, the different inclination can be obtained by a set (not shown in the figures) of removable guide members each defining a guide matrix with different guide angles (each member has parallel guides, while different members have guide angles different from the other members). In this case, the adjustment device for adjusting the spatial orientation of the needle guides consists in a system of connection of the guide member to a support and a set of different guide members.

The electronic device 100 for management of the apparatus, comprising a screen 101 (or other viewing system) available to the surgeon, and an electronic program 102 comprising different operating modules, including an image combination module M1, adapted to combine first images deriving from acquisition of the ultrasonic endocavitary probe 12 with second diagnostic images different from images acquired by the ultrasonic endocavitary probe 12 and relative to the same area where the tumour cells to be destroyed are present, in order to obtain combined third images that can be displayed on the screen 101; a simulation module M2 for simulating destruction of the tumour cells, better described below; and a module M3 relative to the actual intervention for destruction of the tumour cells based on simulations of the module M2 (in practice the module M2 forms a module for planning the subsequent intervention).

With reference to the module M1, the second diagnostic images are for example images relative to a magnetic resonance, preferably in high resolution, concerning a volume of the patient's body in which the tumour area is present. In this example it is a magnetic resonance of a volume around the patient's prostate carried out previously to the moment of treatment in which the apparatus according to the invention is used.

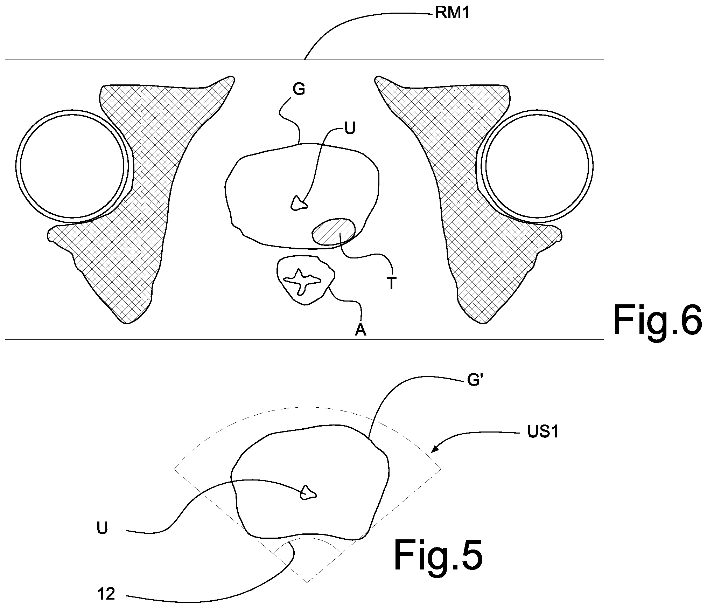

For example, figure 6 shows an image RM1 relative to the transverse plane taken during a magnetic resonance examination relating to a volume around the prostate (analogous images are present relative to the sagittal and coronal planes, in order to complete the three-dimensional image of the magnetic resonance volume, not shown here in order not to weigh down the description and the figures). Alternatively, or in parallel, it is possible to have also a three-dimensional magnetic resonance image relative to the above-mentioned volume.

In these images the prostate G, the urethra U, the rectum A, and the tumour T can be seen, in addition to other anatomical structures of the patient.

In this image RM1 (and in the other two correlated magnetic resonance images), and/or in the 3D image, the tumour area has been identified and in the image (namely in the files showing the images) a graphic circumscription has been provided (by means of an electronic program associated with the magnetic resonance machine or machine for examination of the magnetic resonance results) known as "segmentation". For example, the doctor can follow the profile of the tumour in the transverse plane with an outline figure H1 closed in the transverse plane (which therefore defines a volumetric figure including the tumour that follows the outlines thereof, namely a figure corresponding to the form of the tumour visible in the image). This circumscription/segmentation operation that follows the profile of the tumour (namely of the tumour area) is repeated also in the two images referring to the other two planes and therefore assumes the connotation of a volume defined and spatially positioned in known coordinates in the set of volumetric images provided by the magnetic resonance.

The magnetic resonance images are loaded in the electronic program 102 and are therefore available to the surgeon. It should be noted that, in alternative embodiments, the circumscription operation H1 of the tumour described above can be carried out within the electronic program 102.

The surgeon acquires the first ultrasonic images relative to the same sagittal, coronal and transverse planes (and/or an equivalent first 3D image) by means of the transrectal probe 12 of a volume of the patient's body around the prostate. For simplicity and coherently with what was shown for the magnetic resonance images, only the image US1 relative to the transverse plane is shown.

In said first images the prostate G (but not the tumour area on it), and other anatomical structures of the patient such as, for example, the urethra U can be seen. The position (namely spatial coordinates) of the volume referred to by these three images are known since the position of the ultrasonic probe 12 in a given reference system associated with the first support system 17A is known.

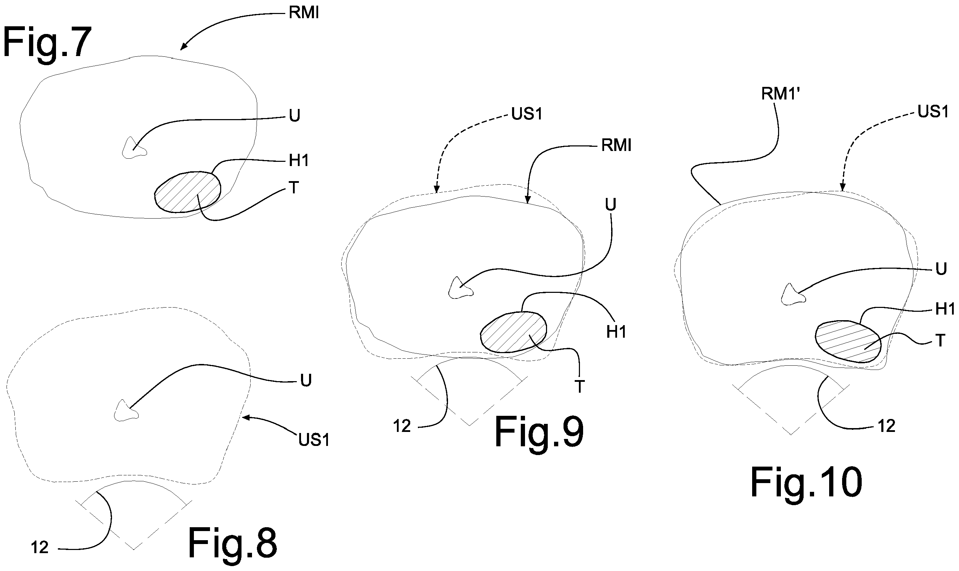

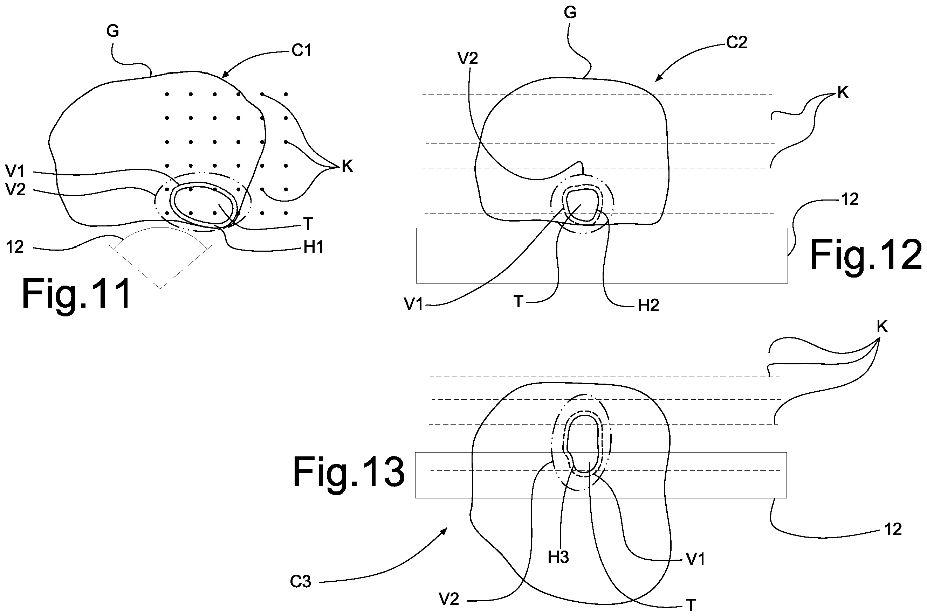

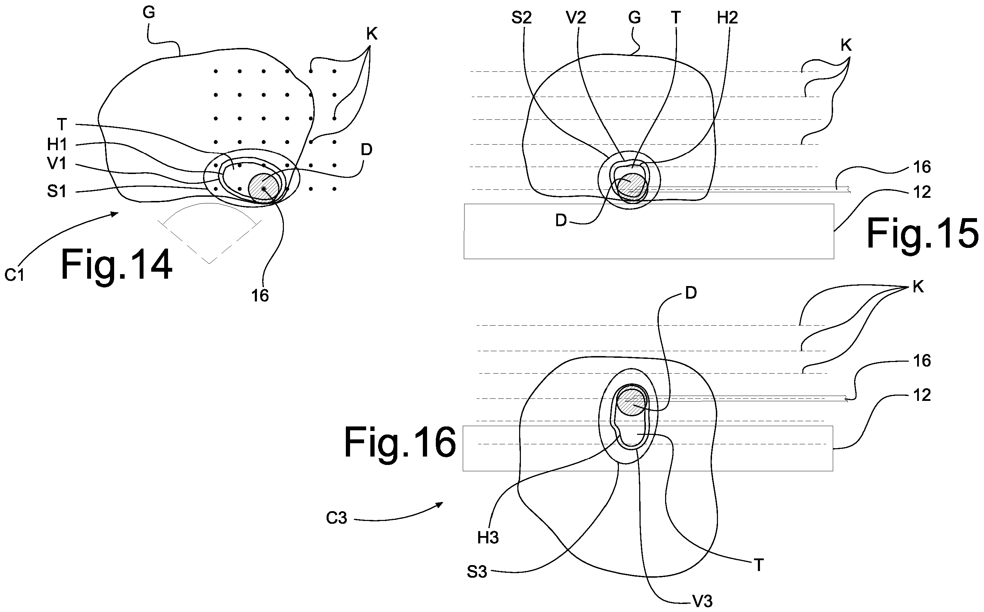

At this point, the module M1 of the electronic program combines the three first images US1 with the respective second images RM1 (by corresponding planes) according to the fusion technique, known per se, which allows corresponding third combined images C1, C2 and C3 to be obtained (by corresponding planes and/or an equivalent third combined 3D image). In said third images, the prostate, the tumour area and the relative outline figures H1-H3, which highlight it in the images, can be seen. These third images C1-C3 are shown on the screen 101 and correspond to real-time images, since the endocavitary probe is inserted in the rectum of the patient. In particular the third images C1 (in practice RM1 + US1) relative to the transverse plane PT are shown in figures 10, 11 and 14, the third images C2 relative to the sagittal or median plane PM are shown in figures 12 and 15, and the third images C3 relative to the frontal/coronal plane PF are shown in figures 13 and 16.

For example, figures 7, 8, 9 and 10 show the process of combination or fusion of the images US1 and RM1. The images are superimposed, causing the structure of the urethra U and the outlines of the prostate gland G, for example, to coincide as far as possible. Since the magnetic resonance image was taken at a different time, whereas the transrectal ultrasound scan is in progress and the probe dilates the anus and the adjacent structures, the outlines of the two prostatic glands will not perfectly correspond. The electronic program tries to minimize these differences, using combination or fusion techniques defined as static combination or elastic combination, according to methods known in the literature.

The static combination in practice entails a superimposition of the two images with a relative positioning based for example on a logic of optimization of the relative distance of said anatomical structures highlighted in the two images.

The combination with deformation entails, for example, an adaptation of the magnetic resonance image to the ultrasound image by means of deformation/stretching/roto-translation of the magnetic resonance image. For example, figure 9 shows the simple superimposition of US1 and RM1, while figure 10 shows the subsequent combination phase in which the image RM1 is deformed into RM1' by the operator by means of the electronic program to obtain an elastic combination of the two images RM1 and US1, namely a deformation in which the operator adapts as far as possible the magnetic resonance image to the first ultrasound image US1 taken by the probe 12.

At this point the electronic program has created the third three-dimensional image, which is illustrated in the three third two-dimensional images C1, C2 and C3 which substantially include a real-time view of the prostate taken by the ultrasonic probe 12 but which also shows the tumour area T, namely, the electronic program 102 allows the surgeon to identify the intervention area, since the tumour is clearly visible from the outlines marked H1-H3 (see figures 11-13).

In practice the third image (third images) can take various forms. For example, it can be an image like C1 in figures 11-13 in which an image transformed by the superimposition of US1 and RM1 can be seen. In another example, the third image can be represented by the first ultrasound image US1 showing the outline (obtained by segmentation) of the tumour area effected in RM1 and positioned in US1 by means of the combination (fusion) operation described above. In other examples there may be, for example, US1 showing RM1 (with the circumscribed tumour area) in transparency over US1 (or the contrary, namely RM1 in transparency). Other combinations are obviously possible.

Once the combination has been obtained, the surgeon activates the simulation/planning module M2.

The surgeon has to decide how much of the prostate to ablate by means of the laser radiation produced by the heads 15, ensuring that anatomical structures to be safeguarded are not affected.

To do this, the electronic program 101 allows the highlighting of an area around the tumour area identified by H1 that defines a margin for the destructive intervention by the laser emitted from the ablation heads 15 which is larger than the tumour itself, in order to guarantee complete destruction of all the tumour cells. For example, said area, also called ablation margin, is defined by a volume spaced from the outline H1-H3 of the tumour for example by a given distance such as, for example, 2 to 5 mm, according to the surgeon's directions. In practical terms, said intervention area is, for each image C1-C3, a closed form V1-V3 which includes the tumour area T, for example a figure that follows the shape of the tumour and is at a given distance from it (in practice a figure in offset with respect to the outline of the tumour area T). In other examples the form of the intervention margin can be delimited differently, for example by means of a well-defined geometric figure. The ablation will be performed on all the area within said volumetric form V1-V3.

Furthermore, the electronic program 102 allows the definition of a safety margin S1-S3, namely a form that surrounds (in each of the three flat images C1-C3, or in the equivalent three-dimensional image) the intervention margin V1-V3 in order to define a safety area outside which no ablation must be performed, to guarantee the anatomical structures surrounding the tumour.

Also, in this case said safety margin S1-S3 can be defined by an offset of the tumour area, or by a well-defined form. In the attached figures, said safety margin S1-S3 is shown for example by an ellipsoidal volume.

It should be noted that, in other embodiments, both the forms defining the intervention area V1-V3 and the forms defining the safety area S1-S3 can be designed directly on the magnetic resonance images Rm1-Rm3 before the combination phase, subsequently transferring them to the combined images C1-C3.

The electronic program 102 displays in the third combined images C1-C3 also the trajectories K for the ablation heads 15. In fact, as said, the position of the trajectories K is known with respect to the ultrasonic volume detected by the probe 12 (the position of the probe 12 and therefore of the ultrasound volume detected by it, and the orientation of the guide member 21 are known in the same reference system). In this example, the trajectories K are rectilinear and orthogonal to the transverse plane, hence in figures C1 said trajectories are shown, for simplicity, by dots or puntiform areas arranged according to a matrix, whereas in figures C2 and C3 by parallel lines (indicated by broken lines).

It is therefore possible to virtually position the laser emission heads 15 around or in the intervention area defined by the form V1-V3. For example, figures 14-16 schematize a needle 16 carrying at its end the optical fibre with the emission head 15.

By means of the simulation/planning module M2 of the electronic program, the surgeon virtually positions one or more heads in the areas he considers most suitable for performing the ablation and virtually sets the treatment parameters that determine the ablation.

Said parameters provide for indication of the power of the laser light emitted by the laser light source (for each emission head or for all the heads), the quantity or dose of energy of laser light emitted for each head, the number of any pullbacks of the emission head from the treatment area outwards and the length of said pullbacks.

Said parameters define a volume that can be irradiated (ablation volume or area) D of laser light, or ablation volume, for each image C1, C2, C3 displayed on the screen. In this example, said ablation volume is schematized by means of a spherical volume and therefore, in the various figures C1-C3, by circles. The form of the ablation volume can be different from the one indicated, and can also be a function, for example, of any pullbacks set.

The heads can be arranged and act all together in the area to be treated or be inserted according to a given sequence (and act according to a given sequence). Obviously also the number and the positions of said heads contribute to forming the ablation process parameters.

When the surgeon views the ablation volume in the various images, he can see whether the setting of the treatment parameters is sufficient to perform an ablation of the whole treatment area V1-V3 without overstepping the safety area S1-S3.

If not, the surgeon changes the treatment parameters, for example changing position and/or number of the emission heads, laser power, etc. and carries out a new simulation.

Simulation of the effect of one or more ablation heads 15 in the area to be treated can be carried out in various ways.

A first method provides for a simulation "in line", namely the surgeon sets the parameters indicated above and launches a simulation with these parameters. The electronic program calculates directly, by means of appropriate algorithms (described in further detail below), the ablation area D and displays it on the screen.

To speed up the intervention times without having to wait for the electronic program to perform the calculation, a second method provides for the use of a database of previous simulations. In practice this database contains the results of simulations for every possible combination of the input parameters specified above, given the specific equipment used, i.e. the type of ablation heads, the number of guides and their mutual positioning, namely:

- number of optical fibres carrying the laser ablation light
- mutual positioning of the ends of said optical fibres (needles carrying the optical fibres) defining the ablation heads
- energy dose applied by each ablation head
- power of the laser source
- pullback actions
- pullback length/distance

In practice a computer has calculated a very high number of simulations for a very wide range of combinations, which substantially comprise all the possible cases of ablation with given laser sources, given ablation heads and given guide members for the needles with the optical fibres. Once the surgeon has positioned the ablation heads 15 on the screen along the virtual trajectories K, and hypothesized all the values of the above-mentioned parameters, he/she only has to recall the desired simulation from the database for immediate display of the result, without waiting for the calculation to be performed by the simulation.

The simulations are obtained using a mathematical model based on the modified Pennes' equation:

$$c\rho\frac{\partial T}{\partial t} = \nabla \cdot (\kappa \nabla(T)) + Q_{laser} + Q_{perf} + Q_e + Q_{met}$$

where T is the tissue temperature in Kelvin, $\rho$ is the tissue density [kg / cm3], c is the specific heat of the tissue [J·kg$^{-1}$·K$^{-1}$], $\kappa$ s the thermal conductivity of the tissue [W·m$^{-1}$·K$^{-1}$], Qlaser is the energy added externally per unit of volume, Qperf is the transfer of heat deriving from the blood perfusion, Qe is the exchange of heat due to the vaporization (boiling) of the water and Qmet is the metabolic heat exchange (which is negligible in the laser ablation and removed from the calculations).

The optical field can be calculated via the scattering equation which applies to turbid media where the scattering coefficient is not negligible like the tissues or via modelling of the Gaussian field at the output of a flat-tip optical fibre. If the emitter is of complex type, for example sidefire or ring fire, the spatial optical field will have to be remodelled each time taking account of the principle of conservation of energy in a vacuum.

At each instant of time, the optical distribution coming out of the optical fibre is calculated; the Pennes' equation is used to obtain the thermal field which acts via the Arrhenius equation on tissue denaturation. The tissues denatured by the heat change their optical and thermal properties and the blood perfusion is altered due to coagulation of the tissues. This simulation scheme takes account of the modifications induced by the coagulation, updating the optical and thermal parameters of the portions of tissue affected by the denaturation, then beginning a new cycle for a subsequent instant of time.

The Arrhenius damage model is the following:

$$\Omega = -\ln\left[\frac{c(t)}{c(t_0)}\right] = \int_0^t \frac{\kappa}{\eta} T \cdot \exp\left(\frac{\Delta S}{R}\right) \cdot \exp\left(-\frac{\Delta H}{RT}\right) dt'$$

where $c(t_0)$ is the concentration of the initial cells and $c(t)$ the concentration of the vital cells at the instant t. $\Delta S$ : Entropy of activation [cal mol$^{-1}$ K$^{-1}$], $\Delta H$ : Enthalpy of activation [kcal mol$^{-1}$], $R$ : gas constant, T: temperature.

As $\Omega$ varies, the following denatured tissue values are obtained as a percentage with respect to the initial condition (native tissue):

$$\Omega = 3 - 95\% \text{ denatured tissue;}$$

$$\Omega = 2 - 86\% \text{ denatured tissue;}$$

$$\Omega = 1 - 63\% \text{ denatured tissue;}$$

$$\Omega = 0.7 - 50\% \text{ denatured tissue;}$$

$$\Omega = 0.6 - 46\% \text{ denatured tissue.}$$

Depending on whether a reference value of $\Omega$ is chosen for the coagulated tissue, for example $\Omega = 1$, the entire time simulation of a volume will give rise to a set of closed surfaces delimiting the denatured part from the non-denatured part.

It is therefore clear that also the selected value of the Arrhenius damage $\Omega$ must be included among the various parameters listed above at the base of the simulations.

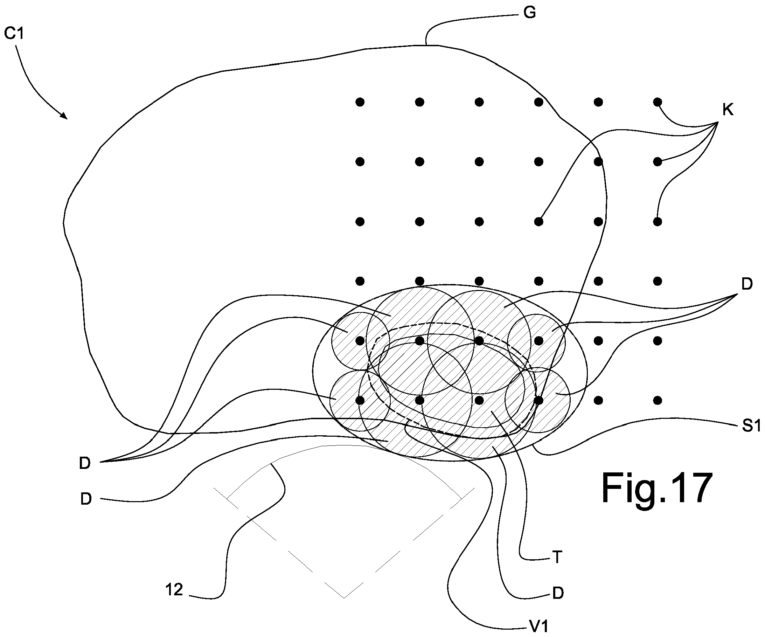

When the simulation (direct, or taken from the database) has given a positive result, namely it shows the treatment area V1-V3 completely superimposed on the ablation areas of the ablation heads, for example as in the case of figure 17 (the same check must obviously be carried out also in the other two images C2 and C3), the surgeon uses the parameters that have given a positive result in the simulation module M2 and carries out the real treatment, introducing the needles 16 with the heads 15 in the desired positions through the perineum, powering the laser with the desired power and operating the desired movements of the needles, following the indications of the simulation.

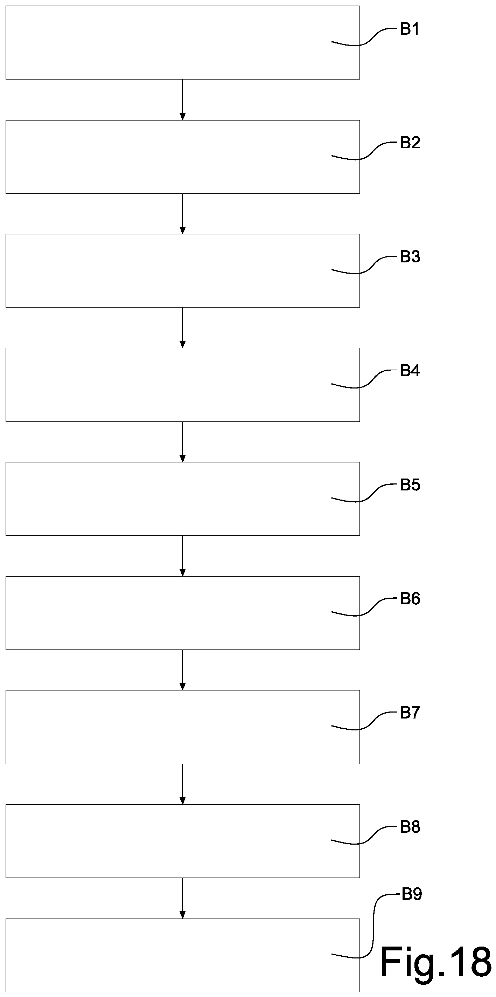

Figure 18 shows a block diagram of the processing method of diagnostic images now described, to simulate the emission of tumour cell destructive laser light.

In particular a first block B1 is provided relative to acquisition of the second three-dimensional magnetic resonance image (namely two or three two-dimensional images that can be combined to produce a three-dimensional image), and in said second image the prostate is present and the tumour area to be eliminated is visible, as in figure 6.

The method also provides a block B2 relative to the acquisition of a first three-dimensional image (namely two or three two-dimensional images that can be combined to produce a three-dimensional image), coming from the ultrasonic probe, in which the prostate is visible, as in figure 5.

Therefore, there is a block B3 associating with the first three-dimensional image a virtual positioning structure for a tumour cell destructive radiation virtual emission head (the virtualization of the guide trajectories K on said first image). Said association is not necessarily shown on the screen in this phase.

A block B4 is relative to the combination of the first and second three-dimensional image in a third three-dimensional combined image (namely two or three two-dimensional images that can be combined to produce a three-dimensional image) C1-C3, which shows the prostate and the tumour area. In said combination, the virtual positioning structure of the virtual emission head is associated with the new third combined image C1-C3 as in figures 11-17 (trajectories K).

A subsequent block B5 provides for definition, on the third combined image C1-C3, of the intervention area V1-V3 where the laser light is to be emitted for ablation of the tumour cells, as in figures 11-17.

A possible block B6 provides for the definition, on the third combined image C1-C3, also of a safety area S1-S3 which circumscribes the intervention area, said circumscription also being visible in the third image C1-C3; said safety area must not be overstepped by the damage caused to the tissues by the ablation heads.

A subsequent block B7 is relative to the virtual positioning, on the third combined image C1-C3, of one or more virtual emission heads in the area of the virtual positioning structure so as to virtually emit the destructive radiation in the intervention area V1-V3, as in figures 14-16.

A possible block B8 concerns display of the volume(s) D that can be irradiated by the virtual emission head virtually positioned in the intervention area V1.

A block B9 is relative to verification that the volume D that can be irradiated by the virtual emission head(s) covers the whole intervention area V1-V3 and that it does not reach beyond the safety area S1-S3.

It is understood that what is illustrated here represents only possible non-limiting embodiments of the invention, which can vary in their forms and arrangements without departing from the concept underlying the invention. The presence of any reference numbers in the attached claims has the sole purpose of facilitating the reading thereof in the light of the preceding description and the attached drawings and does not in any way limit the protective scope thereof.

**Claims**

1. Apparatus (10) for the emission of tumor cell destructive radiation, comprising

an ultrasonic probe (12) configured to acquire at least a first image (US1) relating to a spatial volume where the tumor cells to be destroyed are present,

a first support system (17), configured to support said ultrasonic probe (12) in the area to be investigated, the position of said probe in said first support system (17) being known,

at least one radiation emission head (15) configured to emit destructive radiation being destructive for tumor cell,

a second support system (20), configured to support and guide said at least one emission head (15), along a given trajectory (K), in or in the vicinity of the area with the tumor cells to be destroyed, said second support system (20) being in spatial relationship with said first support system (17) whereby the position of said trajectory (K) defined by said second guide system (20) with respect to said ultrasonic probe (12) and with respect to the spatial volume investigated by said ultrasonic probe is known,

an electronic device (100) for managing the apparatus, comprising a screen (101) and an electronic program (102) adapted to combine at least one said first image (US1) deriving from the acquisition of said ultrasonic probe (12), with at least one second diagnostic image (RM1) different from the images acquired by said ultrasonic probe (12) and relating to the same area where the tumor cells to be destroyed are present, to obtain at least a third combined image (C1, C2, C3), and wherein the at least one third combined image is adapted to be displayed on said screen (101),

wherein said electronic program (102) comprises a simulation module (M2) for simulating destruction of tumor cells which provides for the operations of

spatial definition, on at least said third combined image (C1, C2, C3) displayed on the screen (102), of the intervention area (V1-V3) where to perform the destructive treatment of tumor cells,

displaying, on said at least a third combined image (C1, C2, C3) displayed on the screen, of a simulation of the position of at least part of said given trajectory (K) defined by said second support system (20), so that it is possible to virtually position at least one said emission head (15) in said intervention area (V1-V3),

virtual positioning of at least one said emission head (15) in said intervention area (V1-V3) displayed on the screen (102) in said at least one third combined image (C1, C2, C3),

displaying, on said at least a third combined image (C1, C2, C3) displayed on the screen, of a simulation of the volume that can be irradiated by said at least one emission head (15) virtually positioned in said intervention area (V1-V3),

wherein said simulation module provides a database containing the results of previous simulations carried out on the basis of the following treatment parameters: number of the emission heads (15), the mutual positions of the emission heads (15) and the energy dose applied by each emission head (15), wherein the results of a previous simulation carried out on the basis of the same parameters are retrievable from said database by the surgeon, and wherein the electronic program comprises a module for the destructive treatment of the area with the tumor cells configured to set treatment parameters corresponding to the virtual treatment parameters set on the apparatus during operation of simulation module, said treatment parameters comprising said number of the emission heads (15), said mutual positions of the emission heads (15), and said energy dose applied by each emission head (15).

2. Apparatus according to claim 1, wherein said at least one second image (RM1) is of a different type from images deriving from ultrasonic probes and more preferably is an image deriving from a magnetic resonance, preferably in high definition.

3. Apparatus according to one or more of the preceding claims, wherein said ultrasonic probe (12) is an endocavitary ultrasonic probe, preferably of the transrectal type.

4. Apparatus according to one or more of the preceding claims, wherein said processing program provides that, before combining said at least one first and at least one second image (RM1), said second image (RM1) shows a circumscription of the area occupied by the tumor cells, whereby said circumscription of the area with the tumor cells is also found in said at least a third combined image (C1, C2, C3); preferably said processing program provides for a circumscription operation of the area with the tumor cells before combining said at least one first and at least one second image (RM1).

5. Apparatus according to one or more of the preceding claims, wherein the size of the volume that can be irradiated by said at least one emission head (15) virtually positioned in said intervention area (V1-V3) is a function of one or more of the following parameters: power of the emitted radiation, the quantity or dose of energy of the emitted radiation, the possible movement of the at least one emission head (15) during the emission step, the length of said possible movement, the Arrhenius damage value.

6. Apparatus according to one or more of the preceding claims, wherein said module for the destructive treatment is configured to set also the following parameters: power of the radiation emitted by the at least an emission head (15), the possible movement of the at least one emission head (15) during the emission step, the length of said possible movement.

7. Apparatus according to one or more of the preceding claims, wherein at least one emission head (15) for emitting destructive radiation of tumor cells is an organ capable of emitting a laser beam; preferably the end of an optical fiber.

8. Apparatus according to one or more of the preceding claims, wherein at least one tumor cell destructive radiation emission head (15) is arranged within a guide needle movable along said given trajectory (K) defined by said second support system (20).

9. Apparatus according to one or more of the preceding claims, wherein said ultrasonic probe (12) comprises an oblong body (12A), with a convex curved outer surface, extending along a longitudinal development of said body, wherein a plurality of ultrasonic sensors (12B, 12B', 12B'') facing the curved surface are provided on the body to emit and receive ultrasonic waves; preferably said curved surface of said ultrasonic probe (12) is substantially cylindrical and has a longitudinal axis (Q) parallel to the longitudinal development of the oblong body; preferably, the ultrasonic sensors of the probe (12) are arranged according to at least one rectilinear curtain, parallel to the axis of the probe (12) and at least one curvilinear curtain, lying on a circumference on a plane orthogonal to the axis (Q) of the probe (12).

10. Apparatus according to claim 9, wherein said ultrasonic sensors (12B) are arranged according to a two-dimensional matrix; preferably said two-dimensional matrix having a first dimension parallel to the longitudinal extension of the body of the ultrasonic probe (12) and a second dimension substantially orthogonal to the first dimension; preferably said curved surface of said ultrasonic probe (12) is substantially cylindrical and has a longitudinal axis parallel to the longitudinal extension of the oblong body, and wherein the ultrasonic sensors (12B) are aligned according to a plurality of lines parallel to each other and parallel to the longitudinal axis of the cylindrical surface.

11. Apparatus according to claim 10, comprising a device for controlling the switching of the ultrasonic sensors of said ultrasonic probe (12); preferably said device for controlling the switching of the ultrasonic sensors (12B) being configured to sequentially activate ultrasonic sensors belonging to consecutive lines parallel to the longitudinal axis (Q) of the cylindrical surface, to acquire a sequence of ultrasound images according to a plurality of angularly offset scanning planes passing through the longitudinal axis of the cylindrical surface and angularly offset from each other; preferably said device for controlling the switching of the ultrasonic sensors being configured to sequentially activate ultrasonic sensors belonging to consecutive circumferential lines, to acquire ultrasound images according to a plurality of scanning planes orthogonal to the longitudinal axis of the cylindrical surface and offset along said longitudinal axis; preferably said device for controlling the switching of the ultrasonic sensors being configured to simultaneously activate at least one linear array of ultrasonic sensors angularly offset from each other around the longitudinal axis and linearly along the longitudinal axis of the cylindrical surface to acquire an image according to an oblique plane with respect to the longitudinal axis of the cylindrical surface.

12. Apparatus according to one or more of the preceding claims, wherein

- said database of previous simulations is carried out also on the basis of one or more of the following parameters: power of the source of the ablation radiation, possible presence of pullback actions, with the possible definition of the pullback length/distance.

13. Method of processing diagnostic images of a body volume, including at least a first image (US1) acquired by an ultrasonic probe (12) and at least a second image (RM1) acquired by an imaging technique different from that acquired by said ultrasonic probe (12), to simulate destructive treatment of tumor cells by means of simulation of emission of destructive radiation being destructive for tumor cells, wherein said method is actuated by means of an electronic program comprising a simulation module (M2) for simulating destruction of tumor cells and a module (M3) for the destructive treatment of the area with the tumor cell, said method comprising

- acquiring at least one said second image (RM1) showing the area with the tumor cells to be treated,
- acquiring at least one said first image (US1),
- associating to said at least one first image (US1) a virtual positioning structure for at least one virtual head of emission of destructive radiation of tumor cells,
- combining said at least one said first image (US1) with said at least one second image (RM1) to produce at least a third combined image (C1, C2, C3), showing said area with the tumor cells and said virtual structure for positioning the virtual emission head,
- in said third combined image (C1, C2, C3), a form of circumscription of the intervention area (V1-V3) being visible where the destructive radiation of the tumor cells is emitted,
- on said third combined image, virtually positioning said virtual emission head on said virtual positioning structure so as to virtually emit the destructive radiation in said intervention area (V1-V3),
- verifying that the volume irradiated by said at least one virtual emission head covers the entire intervention area (V1-V3),

wherein on said third combined image (C1, C2, C3), the method provides for displaying said volume that can be irradiated by said at least one virtual emission head virtually positioned in said intervention area (V1-V3), wherein said simulation module (M2) provides a database containing the results of previous simulations carried out on the basis of the following treatment parameters: number of the emission heads, the mutual positions of the emission heads and the energy dose applied by each emission head, wherein the results of a previous simulation carried out on the basis of the same parameters are retrievable from said database by the surgeon, and wherein said module (M3) for the destructive treatment of the area with the tumor cells is configured to set treatment parameters corresponding to virtual treatment parameters set on the apparatus during operation of simulation module, said virtual treatment parameters comprising said number of the emission heads, said mutual positions of the emission heads, and said energy dose applied by each emission head.

14. Method of processing diagnostic images according to one or more of the preceding claims, wherein, on said third combined image (C1, C2, C3), a form of circumscription of a safety area that surrounds the intervention area (V1-V3) is visible.

15. Method of processing diagnostic images according to one or more of the preceding claims, wherein said virtual positioning structure is positioned in said at least one image by means of a unique positioning relationship, so that given said image said positioning structure can only assume the position defined by said unique positioning relationship.

**Patentansprüche**

1. Vorrichtung zur Emission von Strahlung zur Tumor-Zellzerstörung mit

einer Ultraschall-Sonde (12), die ausgebildet ist, um ein erstes Bild (US1) aufzunehmen, das sich auf das räumliche Volumen bezieht, in dem die zu zerstörenden Zellen vorhanden sind, einem ersten Trägersystemen (17), das ausgebildet ist, um die Ultraschall-Sonde (12) in dem zu untersuchenden Bereich zu tragen, wobei die Position der Sonde in dem ersten Trägersystem (17) bekannt ist, mindestens einem Strahlungs-Emissionskopf (15), der ausgebildet ist, um zerstörerische Strahlung zu emittieren, die zerstörerisch für die Tumorzellen ist, einem zweiten Trägersystem (20), das ausgebildet ist, um den mindestens einen Emissionskopf (15) entlang einer gegebenen Bahn (K) oder in der Nähe des Bereichs mit den zu zerstörenden Tumorzellen zu tragen und zu führen, wobei das zweite Trägersystem (20) in räumlicher Beziehung zu dem ersten Trägersystem (17) ist, wodurch die Position der Bahn (K), die durch das zweite Führungssystem (20) mit Bezug auf die Ultraschall-Sonde (12) und mit Bezug auf das räumliche Volumen, das durch die Ultraschall-Sonde untersucht wird, bekannt ist,

einer elektronischen Vorrichtung (100) zur Verwaltung der Vorrichtung mit einem Bildschirm (101) und einem elektronischen Programm (102), das ausgebildet ist, um mindestens eines des ersten Bildes (US1), das von der Ermittlung der Ultraschall-Sonde (12) stammt, mit mindestens einem zweiten Diagnose-Bild (RM1) zu kombinieren, das sich von den Bildern unterscheidet, die durch die Ultraschall-Sonde (12) aufgenommen wurden, und sich auf denselben Bereich beziehen, in dem die zu zerstörenden Tumorzellen vorhanden sind, um mindestens ein drittes kombiniertes Bild (C1, C2, C3) zu erhalten, und wobei mindestens ein drittes kombiniertes Bild ausgebildet ist, um auf dem Bildschirm (101) angezeigt zu werden,

wobei das elektronische Programm (102) ein Simulationsmodul (M2) zur Simulation der Zerstörung von Tumorzellen aufweist, das für die Vorgänge der räumlichen Definition auf mindestens dem dritten kombinierten Bild (C1, C2, C3) sorgt, das auf dem Bildschirm (102) des Eingriffsbereichs (V1-V3) angezeigt wird, in dem die zerstörerische Behandlung von Tumorzellen durchzuführen ist,

zur Anzeige von mindestens einem dritten kombinierten Bild (C1, C2, C3), das auf dem Bildschirm angezeigt wird, einer Simulation der Position von mindestens einem Teil der gegebenen Bahn (K), die durch das zweite Trägersystemen (20) definiert wird, sodass es möglich ist, den mindestens einen Emissionskopf (15) in dem Eingriffsbereich (V1-V3) virtuell zu positionieren,

zur virtuellen Positionierung des mindestens einen Emissionskopfes (15) in dem Eingriffsbereich (V1-V3), der auf dem Bildschirm (102) angezeigt wird, in dem mindestens einen dritten kombinierten Bild (C1, C2, C3),

zur Anzeige auf dem mindestens einen dritten kombinierten Bild (C1, C2, C3), das auf dem Bildschirm angezeigt wird, einer Simulation des Volumens, das durch den mindestens einen Emissionskopf (15) bestrahlt werden kann, der virtuell in dem Eingriffsbereich (V1-V3) positioniert ist,

wobei das Simulationsmodul eine Datenbank liefert, die die Ergebnisse von vorher durchgeführten Simulationen auf Basis der folgenden Behandlungsparameter enthält: Anzahl der Emissionsköpfe (15), die gegenseitigen Positionen der Emissionsköpfe (15) und der Energiedosis, die durch jeden Emissionskopf (15) angewendet wurde, wobei die Ergebnisse einer vorherigen Simulation, die aufgrund derselben Parameter durchgeführt wurde, aus der Datenbank durch den Chirurgen auffindbar sind,

und wobei das elektronische Programm ein Modul für die zerstörerische Behandlung des Bereichs mit den Tumorzellen aufweist, das ausgebildet ist, um Behandlungsparameter einzustellen, die virtuellen Behandlungsparametern entsprechen, die in der Vorrichtung während des Betriebs des Simulationsmoduls eingestellt wurden, wobei die Behandlungsparameter die Anzahl der Emissionsköpfe (15), die gegenseitigen Positionen der Emissionsköpfe (15) und die Energiedosis aufweisen, die durch jeden Emissionskopf (15) angewandt wurde.

2. Vorrichtung nach Anspruch 1, wobei mindestens das zweite Bild (RM1) sich von den Bildern unterscheidet, die von den Ultraschall-Sonden abgeleitet werden und vorzugsweise ein Bild ist, das aus Magnetresonanz abgeleitet wird, vorzugsweise in hoher Auflösung.

3. Vorrichtung nach einem oder mehreren der vorstehenden Ansprüche, wobei die Ultraschall-Sonde(12) eine endokavitäre Ultraschall-Sonde ist, vorzugsweise vom transrektalen Typ.

4. Vorrichtung nach einem oder mehreren der vorstehenden Ansprüche, wobei das Verarbeitungsprogramm dafür sorgt, dass vor der Kombination von mindestens einem ersten und einem zweiten Bild (RM1) ,

das zweite Bild (RM1) eine Umschreibung des Bereichs, der von den Tumorzellen besetzt ist, zeigt, wobei die Umschreibung des Bereichs mit den Tumorzellen auch in mindestens einem dritten kombinierten Bild (C1, C2, C3) gefunden wird, wobei vorzugsweise das Verarbeitungsprogramm für einen Vorgang der Umschreibung des Bereichs mit den Tumorzellen sorgt, bevor das mindestens erste und das mindestens zweite Bild (RM1) kombiniert werden.

5. Vorrichtung nach einem oder mehreren der vorstehenden Ansprüche, wobei die Größe des Volumens, das durch den mindestens einen Emissionskopf (15) bestrahlt werden kann, der virtuell in dem Eingriffsbereich (V1-V3) positioniert wird, eine Funktion von einem oder mehreren der folgenden Parameter ist: Leistung der emittierenden Strahlung, der Quantität der Dosis der Energie der emittierenden Strahlung, der möglichen Bewegung des mindestens einen Emissionskopfes (15) während des Emissionsschritts, der Länge der möglichen Bewegung, des Arrhenius-Schadenswertes.

6. Vorrichtung nach einem oder mehreren der vorstehenden Ansprüche, wobei das Modul für die zerstörerische Behandlung ausgebildet ist, um auch die folgenden Parameter einzustellen: Leistung der Strahlung, die durch den Emissionskopf (15) abgegeben wird, die mögliche Bewegung des mindestens einen Emissionskopfes (15) während des Schrittes der Emission, der Länge der möglichen Bewegung.

7. Vorrichtung nach einem oder mehreren der vorstehenden Ansprüche, wobei der mindestens eine Emissionskopf (15) zur Abgabe von zerstörerischer Strahlung von Tumorzellen ein Organ ist, das in der Lage ist, einen Laserstrahl zu emittieren, vorzugsweise das Ende einer optischen Faser.

8. Vorrichtung nach einem oder mehreren der vorstehenden Ansprüche, wobei mindestens der zur Zerstörung von Tumorzellen Strahlung emittierende Kopf (15) innerhalb einer Führungsnadel angeordnet ist, die entlang einer gegebenen Bahn (K) bewegbar ist, die durch das zweite Trägersystem (20) definiert ist.

9. Vorrichtung nach einem oder mehreren der vorstehenden Ansprüche, wobei die Ultraschall-Sonde (12) einen länglichen Körper (12A) mit einer konvexen gekrümmten äußeren Oberfläche aufweist, die sich entlang einer Längsachse des Körpers erstreckt, wobei eine Anzahl von Ultraschall-Sensoren (12 B, 12 B', 12 B"), die zu der gekrümmten Fläche gerichtet sind, auf dem Körper vorgesehen sind, um Ultraschallwellen zu emittieren und zu empfangen, wobei vorzugsweise die gekrümmte Fläche der Ultraschall-Sonde (12) im Wesentlichen zylindrisch ist und eine Längsachse (Q) aufweist, die parallel zu der Längsausdehnung des länglichen Körpers ist, wobei vorzugsweise die Ultraschall-Sensoren der Sonde (12) entsprechend eines rechtwinkligen Vorhangs angeordnet sind, und mindestens einem kurvenlinearen Vorhang, der auf einem Umfang auf einer Ebene orthogonal zu der Achse (Q) der Sonde (12) liegt.

10. Vorrichtung nach Anspruch 9, wobei die Ultraschall-Sensoren (12B) entsprechend einer zweidimensionalen Matrix angeordnet sind, wobei die zweidimensionale Matrix eine erste Abmessung parallel zu der Längsausdehnung des Körpers der Ultraschall-Sonde (12) und eine zweite Abmessung im Wesentlichen orthogonal zu der ersten Abmessung aufweist, wobei vorzugsweise die gekrümmte Fläche der Ultraschall-Sonde (12) im Wesentlichen zylindrisch ist und eine Längsachse parallel zu der Längsausdehnung des länglichen Körpers aufweist, und wobei die Ultraschall-Sensoren (12B) entsprechend einer Anzahl von Linien ausgerichtet sind, die parallel zueinander und parallel zu der Längsachse der zylindrischen Fläche sind.

11. Vorrichtung nach Anspruch 10 mit einer Vorrichtung zum Steuern des Schaltens der Ultraschall-Sensoren der Ultraschall-Sonde (12), wobei vorzugsweise die Vorrichtung zum Steuern des Schaltens der Ultraschall-Sensoren (12B) ausgebildet ist, um sequenziell Ultraschall-Sensoren zu aktivieren, die zu aufeinanderfolgenden Linien parallel zu der Längsachse (Q) der zylindrischen Fläche gehören, um eine Folge von Ultraschall-Bildern entsprechend einer Anzahl von winkelmäßig versetzten abgetasteten Ebenen zu erhalten, die durch die Längsachse der zylindrischen Fläche verlaufen und winkelmäßig voneinander beabstandet sind, wobei vorzugsweise die Vorrichtung zum Steuern des Schaltens der Ultraschall-Sensoren ausgebildet ist, um sequenziell Ultraschall-Sensoren zu aktivieren, die zu aufeinanderfolgenden Umfangslinien gehören, um Ultraschall-Bilder entsprechend einer Anzahl von abgetasteten Ebenen orthogonal zu der der Längsachse der zylindrischen Fläche und entlang der Längsachse versetzt sind, wobei vorzugsweise die Vorrichtung zum Steuern des Schaltens der Ultraschall-Sensoren ausgebildet ist, um gleichzeitig mindestens eine lineare Anordnung von Ultraschall-Sensoren zu aktivieren, die zu der Längsachse voneinander versetzt sind und linear entlang der Längsachse der zylindrischen Fläche angeordnet sind, um ein Bild entsprechend einer schrägen Ebene mit Bezug auf die Längsachse der zylindrischen Fläche zu erhalten.

12. Vorrichtung nach einem oder mehreren der vorstehenden Ansprüche, wobei
die Datenbank der vorherigen Simulationen auch auf Grundlage von einem oder mehreren der folgenden Parameter ausgeführt wird: Leistung der Quelle der Ablations- Strahlung, mögliches vorhanden sein von Rückzug-Aktionen mit der möglichen Definition der Rückzuglänge/des Abstands.

13. Verfahren der Bearbeitung von Diagnose-Bildern eines Körpervolumens mit mindestens einem ersten Bild (US1) das durch eine Ultraschall-Sonde (12) aufgenommen wurde, und mindestens einem zweiten Bild (RM1), das durch eine Bildtechnik unterschiedlich von der durch die Ultraschall-Sonde (12) aufgenommen wurde, um eine Zerstörungs-Behandlung von Tumorzellen mittels einer Simulation der Emission von Zerstörungs-Strahlung, die zerstörend für Tumorzellen ist, zu simulieren, wobei das Verfahren mittels eines elektronischen Programms betätigt wird, das ein Simulationsmodul (M2) zur Simulation der Tumorzellen aufweist, wobei das Verfahren mittels eines elektronischen Programms betätigt wird, das ein Simulationsmodul (2) zur Simulation der Zerstörung von Tumorzellen und ein Modul (3) für die zerstörerische Behandlung des Bereichs mit der Tumorzellen aufweist, wobei das Verfahren aufweist

    - Aufnahme von mindestens dem zweiten Bild (RM1), das den Bereich mit den zu behandelnden Tumorzellen zeigt,

- Aufnahme von mindestens einem ersten Bild (US1),

- Zuordnen an das mindestens eine erste Bild (US1) einer virtuellen Positionsstruktur für mindestens einen virtuellen Emissionskopf für zerstörerische Strahlung von Tumorzellen,

- Kombinieren des mindestens einen ersten Bildes (US1) mit mindestens einem zweiten Bild (RM1) zur Erzeugung von mindestens einem dritten kombinierten Bild (C1, C2, C3), das den Bereich mit den Tumorzellen und eine virtuelle Struktur zur Positionierung des virtuellen Emissionskopfes zeigt,

- wobei in dem dritten kombinierten Bild (C1, C2, C3) eine Form von Umschreibung des Eingriffsbereichs (V1-V3) sichtbar ist, in dem die zerstörerische Strahlung der Tumorzellen emittiert wird,

- wobei in dem dritten kombinierten Bild eine virtuelle Positionierung des virtuellen Emissionskopfes auf der virtuellen Positionsstruktur erfolgt, um so virtuell die zerstörerische Strahlung in dem Eingriffsbereich (V1-V3) zu emittieren,

- Verifizieren, dass das Volumen, das durch den mindestens einen virtuellen Emissionskopf bestrahlt wird, den gesamten Eingriffsbereich (V1-V3) abdeckt,

wobei auf dem dritten kombinierten Bild (C1, C2, C3) das Verfahren für die Darstellung des Volumens sorgt, das durch den mindestens einen virtuellen Emissionskopf bestrahlt wird, der virtuell in dem Eingriffsbereich (V1-V3) positioniert ist,

wobei das Simulationsmodul (M2) eine Datenbank liefert, die die Ergebnisse vorheriger Simulationen enthält, die auf Grundlage der folgenden Behandlungsparameter durchgeführt wurden: Anzahl der Emissionsköpfe, die gegenseitigen Positionen der Emissionsköpfe und die Energiedosis, die durch jeden Emissionskopf angewendet wurde, wobei die Ergebnisse einer vorherigen Simulation, die auf Grundlage derselben Parameter durchgeführt wurden, aus der Datenbank durch den Chirurgen auffindbar sind,

und wobei das Modul (M3) für die zerstörerische Behandlung des Bereichs mit den Tumorzellen ausgebildet ist, um Parameter entsprechend virtuellen Behandlungsparametern einzustellen, die an dem Gerät während des Betriebs des Simulationsmoduls eingestellt wurden, wobei die virtuellen Behandlungsparameter die Anzahl von Emissionsköpfen, die gegenseitigen Positionen der Emissionsköpfe und die Energiedosis aufweisen, die durch jeden Emissionskopf angelegt wurde.

14. Verfahren zur Verarbeitung von Diagnosebildern nach einem oder mehreren der vorstehenden Ansprüche, wobei auf dem dritten kombinierten Bild (C1, C2, C3) eine Form der Umschreibung eines Sicherheitsbereichs sichtbar ist, der den Eingriffsbereich (V1-V3) umgibt.

15. Verfahren zur Verarbeitung von Diagnosebildern nach einem oder mehreren der vorstehenden Ansprüche, wobei die virtuelle Positionsstruktur in mindestens einem Bild mittels einer eindeutigen Positionsbeziehung positioniert wird, sodass in dem gegebenen Bild die Positionsstruktur nur die Position einnehmen kann, die durch die eindeutige Positionsbeziehung definiert ist.

**Revendications**

1. Appareil (10) pour l'émission d'une radiation destructrice de cellules tumorales, comprenant

une sonde ultrasonique (12) configurée pour acquérir au moins une première image (US1) relative à un volume spatial où sont présentes les cellules tumorales à détruire,

un premier système de support (17), configuré pour supporter ladite sonde ultrasonique (12) dans la zone à analyser, la position de ladite sonde dans ledit premier système de support (17) étant connue,

au moins une tête d'émission de radiations (15), configurée pour émettre des radiations destructrices des cellules tumorales,

un deuxième système de support (20), configuré pour supporter et guider ladite ou lesdites tête(s) d'émission (15) le long d'une trajectoire donnée (K), dans ou à proximité de la zone avec les cellules tumorales à détruire, ledit deuxième système de support (20) étant en relation spatiale avec ledit premier système de support (17), la position de ladite trajectoire (K) définie par ledit deuxième système de guidage (20) par rapport à ladite sonde ultrasonique (12) et au volume spatial analysé par ladite sonde ultrasonique étant connue,

un dispositif électronique (100) pour piloter l'appareil, comprenant un écran (101) et un programme électronique (102) apte à combiner ladite ou lesdites première(s) image(s) (US1) dérivant de l'acquisition de ladite sonde ultrasonique (12), avec au moins une deuxième image de diagnostic (RM1) différente des images acquises par ladite sonde ultrasonique (12) et relative à la même zone où les cellules tumorales à détruire sont présentes, pour obtenir au moins une troisième image combinée (C1, C2, C3), ladite ou lesdites troisième(s) image(s) combinée(s) étant apte(s) à être affichée(s) sur ledit écran (101),

dans lequel ledit programme électronique (102) comprend un module de simulation (M2) pour simuler la destruction des cellules tumorales, qui prévoit les opérations de

définition spatiale, sur ladite ou lesdites troisième(s) image(s) combinée(s) (C1, C2, C3) affichée(s) sur l'écran (102), de la zone d'intervention (V1-V3) où doit être pratiqué le traitement destructif des cellules tumorales,

d'affichage, sur ladite ou lesdites troisième(s) image(s) combinée(s) (C1, C2, C3) affichée(s) sur l'écran, d'une simulation de la position d'au moins une partie de ladite trajectoire donnée (K) définie par ledit deuxième système de support (20), de sorte qu'il soit possible de positionner virtuellement ladite ou lesdites tête(s) d'émission (15) dans ladite zone d'intervention (V1-V3),

de positionnement virtuel de ladite ou desdites têtes d'émission (15) dans ladite zone d'intervention (V1-V3) affichée sur l'écran (102) dans ladite ou lesdite(s) troisième(s) image(s) combinée(s) (C1, C2, C3),

d'affichage, sur ladite ou lesdites troisième(s) image(s) combinée(s) (C1, C2, C3) affichée(s) sur l'écran, d'une simulation du volume qui peut être irradié par ladite ou lesdites tête(s) d'émission (15) positionnée(s) virtuellement dans ladite zone d'intervention (V1-V3),

dans lequel ledit module de simulation prévoit une base de données contenant les résultats de simulations préalables effectuées sur la base des paramètres de traitement suivants : le nombre de têtes d'émission (15), les positions respectives des têtes d'émission (15) et la dose d'énergie appliquée par chaque tête d'émission (15), les résultats d'une simulation préalable effectuée sur la base des mêmes paramètres pouvant être extraits de ladite base de données par le chirurgien,

et dans lequel le programme électronique comprend un module pour le traitement destructif de la zone avec les cellules tumorales, configuré pour définir des paramètres de traitement correspondant aux paramètres de traitement virtuels définis sur l'appareil pendant le fonctionnement du module de simulation, lesdits paramètres de traitement comprenant ledit nombre de têtes d'émission (15), lesdites positions respectives des têtes d'émission (15) et ladite dose d'énergie appliquée par chaque tête d'émission (15).

2. Appareil selon la revendication 1, dans lequel ladite ou lesdites deuxième(s) image(s) (RM1) est d'un type différent parmi des images dérivant de la sonde ultrasonique et plus préférablement, il s'agit d'une image dérivant d'une résonance magnétique, de préférence à haute définition.

3. Appareil selon l'une ou plusieurs des revendications précédentes, dans lequel ladite sonde ultrasonique (12) est une sonde ultrasonique endocavitaire, de préférence du type transrectal.

4. Appareil selon l'une ou plusieurs des revendications précédentes, dans lequel ledit programme de traitement prévoit que, avant de combiner ladite ou lesdites première(s) et ladite ou lesdites deuxième(s) image(s) (RM1), ladite deuxième image (RM1) indique une circonscription de la zone occupée par les cellules tumorales, ladite circonscription de la zone avec les cellules tumorales étant aussi trouvée dans ladite ou lesdites troisième(s) image(s) combinée(s) (C1, C2, C3) ; de préférence ledit programme de traitement prévoit une opération de circonscription de la zone avec les cellules tumorales avec de combiner ladite ou lesdites première(s) et ladite ou lesdites deuxième(s) images (RM1).

5. Appareil selon l'une ou plusieurs des revendications précédentes, dans lequel la taille du volume qui peut être irradié par ladite ou lesdites tête(s) d'émission (15) positionnée(s) virtuellement dans ladite zone d'intervention (V1-V3) est une fonction de l'un ou plusieurs des paramètres suivants : la puissance de la radiation émise, la quantité ou dose d'énergie de la radiation émise, le mouvement possible de ladite ou desdites têtes d'émission (15) durant l'étape d'émission, la longueur dudit mouvement possible, la valeur du dommage Arrhenius.

6. Appareil selon l'une ou plusieurs des revendications précédentes, dans lequel ledit module pour le traitement destructif est configuré pour définir également les paramètres suivants : la puissance de la radiation émise par la ou les tête(s) d'émission (15), le mouvement possible de la ou des têtes d'émission (15) durant l'étape d'émission, la longueur dudit mouvement possible.

7. Appareil selon l'une ou plusieurs des revendications précédentes, dans lequel ladite ou lesdites têtes d'émission (15) pour émettre une radiation destructive des cellules tumorales est/sont un élément capable d'émettre un faisceau laser ; de préférence l'extrémité d'une fibre optique.

8. Appareil selon l'une ou plusieurs des revendications précédentes, dans lequel au moins une tête d'émission de radiations destructives de cellules tumorales (15) est agencée à l'intérieur d'une aiguille de guidage mobile le long de ladite trajectoire donnée (K) définie par ledit deuxième système de support (20).

9. Appareil selon l'une ou plusieurs des revendications précédents, dans lequel ladite sonde ultrasonique (12) comprend un corps oblong (12A), avec une surface externe incurvée de manière convexe s'étendant le long d'une extension longitudinale dudit corps, dans lequel une pluralité de détecteurs d'ultrasons (12B, 12B', 12B") faisant face à la surface incurvée sont prévus sur le corps pour émettre et recevoir des ondes d'ultrasons ; de préférence ladite surface incurvée de ladite sonde ultrasonique (12) est sensiblement cylindrique et a un axe longitudinal (Q) parallèle à l'extension longitudinale du corps oblong ; de préférence, les détecteurs d'ultrasons de la sonde (12) sont agencés suivant au moins un rideau rectiligne parallèle à l'axe de l'échantillon (12) et au moins une rideau curviligne situé sur la circonférence d'un plan perpendiculaire à l'axe (Q) de la sonde (12).

10. Appareil selon la revendication 9, dans lequel lesdits détecteurs d'ultrasons (12B) sont agencés suivant une matrice bidimensionnelle ; ladite matrice bidimensionnelle ayant de préférence une première dimension parallèle à l'extension longitudinale du corps de la sonde ultrasonique (12) et une deuxième dimension sensiblement perpendiculaire à la première dimension ; ladite surface incurvée de ladite sonde ultrasonique (12) est de préférence sensiblement cylindrique et a un axe longitudinal parallèle à l'extension longitudinale du corps oblong, et dans lequel les détecteurs d'ultrasons (12B) sont alignés suivant une pluralité de lignes parallèles les unes aux autres et parallèles à l'axe longitudinal de la surface cylindrique.

11. Appareil selon la revendication 10, comprenant un dispositif pour commander la commutation des détecteurs d'ultrasons de ladite sonde ultrasonique (12) ; ledit dispositif pour commander la commutation des détecteurs d'ultrasons (12B) étant de préférence configuré pour activer séquentiellement les détecteurs d'ultrasons appartenant à des lignes consécutives parallèles à l'axe longitudinal (Q) de la surface cylindrique, pour acquérir une séquence d'images d'ultrasons suivant une pluralité de plans de balayage décalés angulairement passant à travers l'axe longitudinal de la surface cylindrique et décalés angulairement les uns par rapport aux autres ; ledit dispositif pour commander la commutation des détecteurs d'ultrasons étant de préférence configuré pour activer séquentiellement des détecteurs d'ultrasons appartenant à des lignes circonférentielles consécutives, pour acquérir des images d'ultrasons suivant une pluralité de plans de balayage perpendiculaires à l'axe longitudinal de la surface cylindrique et décalés le long dudit axe longitudinal ; ledit dispositif pour commander la commutation des détecteurs d'ultrasons étant de préférence configuré pour activer simultanément au moins un arrangement linéaire de détecteurs d'ultrasons décalés angulairement les uns par rapport aux autres autour de l'axe longitudinal et linéairement le long de l'axe longitudinal de la surface cylindrique pour acquérir une image suivant un plan oblique par rapport à l'axe longitudinal de la surface cylindrique.

12. Appareil selon l'une ou plusieurs des revendications précédentes, dans lequel

   - ladite base de données de simulations préalables est réalisée également sur la base de l'un ou de plusieurs des paramètres suivants : puissance de la source de la radication d'ablation, présence éventuelle d'actions de retour, avec la définition éventuelle de la longueur de retour/distance.

13. Procédé de traitement d'images de diagnostic du volume d'un corps, incluant au moins une première image (US1) acquise par une sonde ultrasonique (12) et au moins une deuxième image (RM1) acquise par une sonde ultrasonique (12) et au moins une deuxième image (RM1) acquise par une technique d'imagerie différente de celle acquise par ladite sonde ultrasonique (12), pour simuler un traitement destructif de cellules tumorales au moyen d'une simulation de l'émission d'une radiation destructive pour des cellules tumorales, dans lequel ce procédé est mis en œuvre par un programme électronique comprenant un module de simulation (M2) pour simuler la destruction des cellules tumorales et un module (M3) pour le traitement destructif de la zone avec les cellules tumorales, ce procédé comprenant les étapes consistant à :

   - acquérir au moins ladite ou lesdites deuxième(s) image(s) (RM1) indiquant la zone avec les cellules tumorales à traiter,
   - acquérir ladite ou desdites première(s) image(s) (US1),
   - associer à ladite ou auxdites première(s) image(s) (US1) une structure de positionnement virtuel pour au moins une tête virtuelle d'émission de radiations destructives de cellules tumorales,
   - combiner ladite ou desdites première(s) image(s) (US1) avec ladite ou lesdites deuxième(s) image(s) (RM1) pour produire au moins une troisième image combinée (C1, C2, C3) indiquant ladite zone avec les cellules tumorales et ladite structure virtuelle pour positionner la tête d'émission virtuelle,
   - une forme de circonscription de la zone d'intervention (V1-V3) étant visible dans ladite troisième image combinée (C1, C2, C3), là où la radiation destructive des cellules tumorales est émise,
   - sur ladite troisième image combinée, positionner virtuellement ladite tête d'émission virtuelle sur ladite structure de positionnement virtuelle, de façon à émettre virtuellement la radiation destructive dans ladite zone d'intervention (V1-V3),

- vérifier que le volume irradié par ladite ou lesdites tête(s) d'émission virtuelle(s) couvre(nt) toute la zone d'intervention (V1-V3),

dans lequel sur ladite troisième image combinée (C1, C2, C3), ce procédé prévoyant l'affichage dudit volume qui peut être irradié par ladite ou lesdites tête(s) d'émission virtuelle(s) positionnée(s) virtuellement dans ladite zone d'intervention (V1-V3),

dans lequel ledit module de simulation (M2) prévoit une base de données contenant les résultats des simulations préalables réalisées sur la base des paramètres de traitement suivants : le nombre de têtes d'émission, les positions respectives des têtes d'émission et la dose d'énergie appliquée par chaque tête d'émission, dans lequel les résultats d'une simulation préalable réalisée sur la base des mêmes paramètres peuvent être extraits à partir de ladite base de données par le chirurgien,

et dans lequel ledit module (M3) pour le traitement destructif de la zone avec les cellules tumorales est configuré pour définir des paramètres de traitement correspondant à des paramètres de traitement virtuel définis sur l'appareil durant le fonctionnement du module de simulation, lesdites paramètres de traitement virtuel comprenant ledit nombre de têtes d'émission, lesdites positions respectives des têtes d'émission et ladite dose d'énergie appliquée par chaque tête d'émission.

14. Procédé de traitement d'images de diagnostic selon l'une ou plusieurs des revendications précédentes, dans lequel, sur ladite troisième image combinée (C1, C2, C3), une forme de circonscription d'une zone de sureté qui entoure la zone d'intervention (V1-V3) est visible.

15. Procédé de traitement d'images de diagnostic selon l'une ou plusieurs des revendications précédentes, dans lequel ladite structure de positionnement virtuel est positionnée dans ladite ou lesdites image(s) au moyen d'une relation de positionnement unique, de façon à ce que, une fois ladite ou lesdites image(s) donnée(s), ladite structure de positionnement puisse adopter seulement la position définie par ladite relation de positionnement unique.

Fig.1

Fig.4

20

22

13
16
15
K

X

K

23

21

M

N

Fig.4A

15A
13
16
15

12

12B

12A

Q

12B''

Q

12B'

12

122B

121A

121B

122B

Y1

Y2

Fig.4B

Fig.2

Fig.3

24

EP 4 346 620 B1

Fig.6

RM1

G

U

T

A

Fig.5

G'

US1

U

12

EP 4 346 620 B1

**Fig.7**

RMI

U

H1

T

**Fig.8**

U

US1

12

US1

RMI

U

H1

T

12

**Fig.9**

RM1'

US1

U

H1

T

12

**Fig.10**

Fig.12

Fig.13

Fig.11

Fig.15

Fig.16

Fig.14

EP 4 346 620 B1

Fig.17

Fig.18

Fig.19

**EP 4 346 620 B1**

**Patent documents cited in the description**

- US 20170020623 A1

- WO 2020212893 A